(19) Europäisches Patentamt
European Patent Office
Office européen des brevets

(11) EP 2 147 979 A1

(12) **EUROPEAN PATENT APPLICATION**

(43) Date of publication:
**27.01.2010 Bulletin 2010/04**

(51) Int Cl.:
***C12Q 1/48*** *(2006.01)*

(21) Application number: **08013340.8**

(22) Date of filing: **24.07.2008**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(71) Applicant: **Max-Planck-Gesellschaft zur Förderung der Wissenschaften e.V.**
**80539 München (DE)**

(72) Inventor: **Rauh, Daniel**
**67549 Worms (DE)**

(74) Representative: **Vossius & Partner**
**Siebertstraße 4**
**81675 München (DE)**

(54) **Fluorescently or spin-labeled kinases for rapid screening and identification of novel kinase inhibitor scaffolds**

(57) The present invention relates to a kinase labeled at an amino acid having a free thiol or amino group, wherein said amino acid is naturally present or introduced in the activation loop of said kinase, with (a) a thiol- or amino-reactive fluorophore sensitive to polarity changes in its environment; or (b) a thiol-reactive spin label, an isotope or an isotope-enriched thiol- or amino-reactive label, such that said fluorophore, spin label, isotope or isotope-enriched label does not inhibit the catalytic activity and does not interfere with the stability of the kinase. The invention furthermore relates to a method of screening for kinase inhibitor, a method of determining the kinetics of ligand binding and/or of dissociation of a kinase inhibitor and a method of generating mutated kinases suitable for the screening of kinase inhibitors using the kinase of the present invention.

EP 2 147 979 A1

## Description

The present invention relates to a kinase labeled at an amino acid having a free thiol or amino group, wherein said amino acid is naturally present or introduced in the activation loop of said kinase, with (a) a thiol- or amino-reactive fluorophore sensitive to polarity changes in its environment; or (b) a thiol-reactive spin label, an isotope or an isotope-enriched thiol- or amino-reactive label, such that said fluorophore, spin label, isotope or isotope-enriched label does not inhibit the catalytic activity and does not interfere with the stability of the kinase. The invention furthermore relates to a method of screening for kinase inhibitors, a method of determining the kinetics of ligand binding and/or dissociation of a kinase inhibitor and a method of generating mutated kinases suitable for screening of kinase inhibitors using the labeled kinase of the present invention.

In this specification, a number of documents including patent applications and manufacturer's manuals are cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

Protein kinases play a critical role in regulating many aspects of cellular function. For this reason, they are widely considered to be among the most attractive targets for therapeutic drug development. To date, strategies for inhibiting kinases have been based primarily on compounds designed to bind directly at the natural substrate (i.e. ATP) binding site. These are known as ATP-competitive inhibitors, also termed Type I inhibitors. Recently, inhibitors which bind exclusively to sites adjacent to the ATP-binding pocket and thereby induce an inactivating conformational change in the protein have been found. These compounds are known as allosteric, or Type III, inhibitors. Allosteric inhibitors which bind to this allosteric site but also extending into the ATP-binding pocket of a kinase are also known and termed Type II inhibitors. Examples for the latter are imatinib (Gleevec), sorafenib (Nexavar) and BIRB-796.

Aberrantly regulated kinases play causative roles in many diseases, and the most common strategy for regulating unwanted kinase activity is the use of ATP competitive (Type I) inhibitors. However, the development of drugs which bind to a single specific kinase has been hampered by the high sequence and structural homology in the ATP binding pocket of all kinases, resulting in the low specificity of such inhibitors. A less-conserved hydrophobic pocket adjacent to the ATP binding site was first identified in p38α MAP kinase (Pargellis et al., 2002) and MEK kinases (Ohren et al., 2004) and found to be an allosteric binding site. Inhibition at this site has since been found in several other kinases including Aurora, EGFR, Src, Abl. Kinases are typically in the active conformation (DFG-in) with the activation loop open and extended, allowing ATP and other molecules to bind. Alternatively, the adjacent allosteric site is only available in the inactive conformation (DFG-out) in which the activation loop shifts conformation and interferes with both ATP binding to the ATP-pocket and substrate binding to the allosteric binding site. Various tight binding inhibitors have recently been developed for p38α which either bind in the allosteric pocket exclusively (Type III) or extend from this pocket into the ATP binding site (Type II). The availability of structural information for the inactive state of these kinases has intensified the search for new drug scaffolds which bind to this site with high affinity and increased specificity. Methods for discriminating between compounds which bind in each site are currently limited (Annis et al., 2004; Vogtherr et al., 2006). Furthermore, rapid and feasible high-throughput screening methods for the identification of Type I, II and III inhibitors are not yet available.

The attachment of fluorophores to proteins is a well-established approach used to detect conformational changes in protein structure in response to ligand binding. In addition to the commercially-available probe *acrylodan-labeled fatty acid binding protein* (ADIFAB; Molecular Probes), which measures the concentration of unbound fatty acids in buffer (Richieri et al., 1999), this approach has been applied to various other proteins including acetylcholine binding protein (Hibbs et al., 2004), interleukin-1β (Yem et al., 1992) and various sugar and amino acid binding proteins (de Lorimier et al., 2002). It emanates from the above discussion that it would be desirable to have versatile means and methods for screening for specific kinase inhibitors. The solution to this technical problem is achieved by providing the embodiments characterized in the claims.

Accordingly, the present invention relates to a kinase labeled at an amino acid having a free thiol or amino group, wherein said amino acid is naturally present or introduced in the activation loop of said kinase, with (a) a thiol- or amino-reactive fluorophore sensitive to polarity changes in its environment; or (b) a thiol-reactive spin label, an isotope or an isotope-enriched thiol- or amino-reactive label, such that said fluorophore, spin label, isotope or isotope-enriched label does not inhibit the catalytic activity and does not interfere with the stability of the kinase.

The term "kinase" is well-known in the art and refers to a type of enzyme that transfers phosphate groups from high-energy donor molecules, such as ATP, to specific target molecules such as proteins. Kinases are classified under the enzyme commission (EC) number 2.7. According to the specificity, protein kinases can be subdivided into serine/threonine kinases (EC 2.7.11, e.g. p38α), tyrosine kinases (EC 2.7.10, e.g. the EGFR kinase domain), histidine kinases (EC 2.7.13), aspartic acid/glutamic acid kinases and mixed kinases (EC 2.7.12) which have more than one specificity (e.g. MEK being specific for serine/threonine and tyrosine).

The modified kinases of the present invention are labeled at an amino acid having a free thiol- and/or amino

group. Amino acids are defined as organic molecules that have a carboxylic and amino functional group. They are the essential building blocks of proteins. Examples of amino acids having a free thiol group are cysteine, belonging to the 20 standard amino acids, and acetyl-cysteine being a non-standard amino acid rarely occurring in natural amino acid sequences. Standard amino acids having a free amino group are lysine, histidine or arginine and amino acids being aromatic amines, such as tryptophan. Pyrrolysine, 5-hydroxylysine or o-aminotyrosine are non-standard amino acids having a free amino group. The amino acids asparagine and glutamine, although having a free amino group, are not suitable in the present invention as they are not reactive to labeling agents and are thus excluded.
Tryptophan is an aromatic amino acid having an amino group in its indole ring. Aromatic amines are weak bases and thus unprotonated at pH 7. However, they can still be modified using a highly reactive reagent such as an isothiocyanate, sulfonyl chloride or acid halide.

Said amino acid to be labeled is located in the activation loop of the kinase. This means that only kinases having an activation loop or a structure equivalent thereto fall within the present invention. The activation loop is a flexible segment near the entrance to the active site which forms the substrate binding cleft of most kinases and can be phosphorylated on one or more amino acids to provide an important regulatory mechanism throughout the protein kinase superfamily (Johnson and Lewis, 2001; Taylor and Radzio-Andzelm, 1994; Johnson et al., 1996). The activation loop consists of several amino acids which form a loop that is flexible in most kinases which begins with a highly-conserved aspartate-phenylalanine-glycine (DFG) motif in the ATP binding site and extends out between the N- and C-lobes of the kinase. The activation loop is a structural component crucial for enzymatic kinase activity. It is part of the substrate binding cleft and contains several amino acid residues which assist in the recognition of specific substrates and also contains serines, threonines or tyrosines which can be phosphorylated. The conformation of the activation loop is believed to be in dynamic equilibrium between the DFG-in (active kinase) and DFG-out (inactive kinase) conformations. Phosphorylation and/or binding of interaction partners (other proteins or DNA) result in a shift of the equilibrium. In the DFG-in conformation, the aspartate contained in the motif is pointed into the ATP binding site and the adjacent phenylalanine is pointed away from the ATP site and into the an adjacent allosteric site. When the conserved DFG motif forming part of the activation loop adopts the in-conformation, ATP-competitive inhibitors (Type I inhibitors) can bind to the kinase. In the DFG-out conformation, the positions of these residues are flipped 180° in orientation. The out-conformation of the activation loop prevents ATP and substrate binding. Compounds causing a conformational change of the activation loop from the in- to the out-conformation are either Type II inhibitors, which bind to the ATP site (hinge region) and extend into the allosteric pocket adjacent to the ATP binding site, or Type III inhibitors, which only bind to the allosteric pocket.
Cysteines which are naturally present in a kinase of interest and are solvent-exposed can be located outside the activation loop or within the activation loop sequence. This equally applies to amino acids having a free amino group.

The modified kinase of the invention is labeled at an amino acid naturally present or introduced into the activation loop. If no suitable amino acid, i.e. one having a free thiol- or amino group, is present in the activation loop, said amino acid can be introduced, i.e. inserted by adding it or by replacing an existing amino acid, by techniques well-known in the art. In any case, it is to be understood for the avoidance of doubt that the amino acid is only labeled after its introduction into the activation loop if it is to be labeled by reaction with labeling reagents. Those techniques comprise site-directed mutagenesis as well as other recombinant, synthetic or semisynthetic techniques. In case a non-standard amino acid is to be introduced into the kinase, an amino acid stretch containing said amino acid may be chemically synthesized and then connected to the remaining part(s) of the kinase which may have been produced recombinantly or synthetically.

The process of labeling involves incubation of the kinase, e.g. the mutated kinase of the invention, (e.g. the kinase with a cysteine introduced in the activation loop), with a thiol- or amino-reactive label under mild conditions resulting in the labeling of said mutated kinase at the desired position in the activation loop. Mild conditions refer to buffer pH (e.g. around pH7 for thiol-reactive probes), ratio of label to kinase, temperature and length of the incubation step (for thiol-reactive probes e.g. 4 °C and overnight in the dark) which are known to the skilled person and provided with instruction manuals of providers of thiol-and amino-reactive probes. Such conditions need to be optimized to slow down the reaction of the chosen thiol- or amino-reactive label to ensure that labeling of said kinase is specific to the desired labeling site. In the case of fluorophore labeling, it is necessary to carry out the incubation in the dark. Increased light exposure results in bleaching of the fluorophore and a less intense fluorescence emission. After labeling, the labeled kinase is preferably concentrated, purified by gel filtration experiments or washed several times with buffer to remove excess unreacted label. The wash buffer is typically the buffer used to store the labeled kinase and may also be the buffer in which the desired measurements are made.

The term "fluorophore" denotes a molecule or functional group within a molecule which absorbs energy such as a photon of a specific wavelength and emits energy, i.e. light at a different (but equally specific) wavelength (fluorescence) immediately upon absorbance (unlike the case in phosphorescence) without the involvement of a chemical reaction (as the case in bioluminescence). Usually the wavelength of the absorbed photon is in the ultraviolet range but can reach also into the infrared range. The wavelength of the emitted light is usually in the visible range. The amount and wavelength of the emitted energy depend primarily on the properties of the fluorophore but may also be influenced

by the chemical environment surrounding the fluorophore. A number of fluorophores are sensitive to changes in their environment. This includes changes in the polarity, charge and/or in the conformation of the molecule they are attached to. Fluorescence occurs when a molecule relaxes to its ground state after being electrically excited which, for commonly used fluorescent compounds that emit photons with energies from the UV to near infrared, happens in the range of between 0.5 and 20 nanoseconds.

The term "thiol- or amino-reactive" denotes the property of a compound, e.g. a fluorophore, to specifically react with free thiol- or amino groups. This is due to a functional group present in said compound which directs a specific reaction with a thiol or amino group. These functional groups may be coupled to molecules such as fluorophores, spin labels or isotope-enriched molecules in order to provide specific labels attachable to free thiol- or amino-groups. Examples for thiol-specific compounds are e.g. haloalkyl compounds such as iodoacetamide, maleimides, Hg-Link™ phenylmercury compounds or TS-link™ reagents (both Invitrogen). Haloalkyl compounds react with thiol or amino groups depending on the pH.

The term "spin label" (SL) denotes a molecule, generally an organic molecule, which possesses an unpaired electron, usually on a nitrogen atom, and has the ability to bind to another molecule. Spin labels are used as tools for probing proteins using EPR spectroscopy. The site-directed spin labeling (SDSL) technique allows one to monitor the conformation and dynamics of a protein. In such examinations, amino acid-specific SLs can be used.

Site-directed spin labeling is a technique for investigating protein local dynamics using electron spin resonance. SDSL is based on the specific reaction of spin labels with amino acids. A spin label built in protein structures can be detected by EPR spectroscopy. In SDSL, sites for attachment of spin labels such as thiol or amino groups, if not naturally present, are introduced into recombinantly expressed proteins by site-directed mutagenesis. Functional groups contained within the spin label determine their specificity. At neutral pH, protein thiol groups specifically react with functional groups such as methanethiosulfonate, maleimide and iodoacetamide, creating a covalent bond with the amino acid cysteine. Spin labels are unique molecular reporters, in that they are paramagnetic, i.e. they contain an unpaired electron. Nitroxide spin labels are widely used for the study of macromolecular structure and dynamics because of their stability and simple EPR signal. The nitroxyl radical (N-O) is usually incorporated into a heterocyclic ring such as pyrrolidine, and the unpaired electron is predominantly localized to the N-O bond. Once incorporated into the protein, a spin label's motions are dictated by its local environment. Because spin labels are exquisitely sensitive to motion, this has profound effects on the EPR spectrum of the spin-label attached to the protein.

The signal arising from an unpaired electron can provide information about the motion, distance, and orientation of unpaired electrons in the sample with respect to each other and to the external magnetic field. For molecules free to move in solution, EPR works on a much faster time-scale than NMR (Nuclear Magnetic Resonance spectroscopy), and so can reveal details of much faster molecular motions, i.e. nanoseconds as opposed to microseconds for NMR. The gyromagnetic ratio of the electron is orders of magnitude larger than of nuclei commonly used in NMR, and so the technique is more sensitive, though it does require spin labeling.

The term "isotope" denotes a chemical species of a chemical element having different atomic mass (mass number) than the most abundant species of said element. Isotopes of an element have nuclei with the same number of protons (the same atomic number) but different numbers of neutrons. Isotopes suitable for EPR or NMR need to have a nonzero nuclear spin. The most common isotopes currently used are $^{1}H$, $^{2}D$, $^{15}N$, $^{13}C$, and $^{31}P$.

The term "isotope-enriched" denotes that a compound, e.g. a thiol- or amino-reactive label has been synthesized using or reacted with an isotope so that said isotope is introduced into said compound. The compound may comprise one or more isotopes of one or more different species.

The label has to be positioned so that it does not inhibit the kinase's catalytic activity and does not interfere with its stability. In the case of a kinase that is isotopically labeled on an amino acids, e.g. a cysteine and produced by growing host organisms expressing the kinase with isotopically labeled amino acid already incorporated into the sequence, inhibition of the activity or interference with the stability of the kinase is unlikely. On the other hand, care also has to be taken when selecting the position in the activation loop where the label is to be introduced. If no suitable amino acid is present at the position of choice, the amino acid present at said position must be replaced with an amino acid containing a free thiol or amino group. Tests of how to evaluate the activity and stability of a kinase prior to and after replacement of an amino acid are well known to the skilled person and include visual inspection of the purified protein, circular dichroism (CD) spectroscopy, crystallization and structure determination, enzyme activity assays, protein melting curves, differential scanning calorimetry and NMR spectroscopy.

In this regard, no inhibition of the catalytic activity is present if at least 90% of the catalytic activity of the kinase are retained, preferably 95%, more preferably 98%. Most preferably, the catalytic activity of the kinase is fully retained. The term "does not inhibit the catalytic activity" is thus, in some embodiments where the catalytic activity amounts to less than 100 %, to be equated with and having the meaning of "does not essentially interfere with the catalytic activity". Regarding stability, the amino acid introduced does not interfere with the essential intramolecular contacts that ensure structural stability of the protein, so that the kinase can carry out the biological function described herein.

To overcome the drawbacks of presently existing screening methods, the present invention involves a labeling

strategy to create fluorescent-tagged kinases which (i) are highly sensitive to the binding of kinase inhibitors, (ii) can be used to measure the kinetics of ligand binding and dissociation in real-time, (iii) can be used to directly measure the Kd of these ligands and (iv) is rapid, robust, reproducible and adaptable to high-throughput screening methods.

In contrast to the prior art and as demonstrated in the appended examples, the present invention provides kinases and screening methods using these kinases which enables for screening for specific inhibitors with a reduced effort and material and as well as a superior reliability. This is essentially achieved by providing a labeling strategy for a kinase such that the label alters its behavior in reaction to changes in its environment caused e.g. by conformational changes in the activation loop of the kinase.

Besides conventional kinase assays for the screening of modulators of kinase activity, various approaches have recently been developed. However, many of these approaches suffer from major drawbacks. For example, Annis et al. (2004) describe an approach using affinity selection- mass spectrometry (AS-MS). This method is described as suitable for high-throughput screening. However, a size exclusion chromatography step has to be applied prior to the examination of each probe which is time-consuming and requires a lot of material.

De Lorimier et al. (2002) describe a family of biosensors based on bacterial proteins binding to small molecule ligands which were modified and labeled with different environmentally sensitive fluorophores. Upon ligand binding, the fluorophores alter their emission wavelength and/or intensity thus indicating the presence and/or concentration of the specific ligand bound to a probe. However, the labeling of kinases and the use of said kinases in the screening for specific inhibitors is neither disclosed nor suggested.

In a preferred embodiment, the kinase is p38α, MEK kinase, CSK, an Aurora kinase, GSK-3β, c-Src, EGFR, Abl, DDR1, LCK or another MAPK.

Mitogen-activated protein (MAP) kinases (EC 2.7.11.24) are serine/threonine-specific protein kinases that respond to extracellular stimuli (mitogens) and regulate various cellular activities, such as gene expression, mitosis, differentiation, and cell survival/apoptosis. Extracellular stimuli lead to activation of a MAP kinase via a signaling cascade ("MAPK cascade") composed of a MAP kinase, MAP kinase kinase (MKK or MAP2K) and MAP kinase kinase kinase (MKKK or MAP3K, EC 2.7.11.25).

A MAP3K that is activated by extracellular stimuli phosphorylates a MAP2K on its serine and/or threonine residues, and then this MAP2K activates a MAP kinase through phosphorylation on its serine and/or tyrosine residues. This MAP kinase signaling cascade has been evolutionarily well-conserved from yeast to mammals.

To date, six distinct groups of MAPKs have been characterized in mammals:

1. extracellular signal-regulated kinases (ERK1, ERK2). The ERK (also known as classical MAP kinases) signaling pathway is preferentially activated in response to growth factors and phorbol ester (a tumor promoter), and regulates cell proliferation and cell differentiation.

2. c-Jun N-terminal kinases (JNKs), (MAPK8, MAPK9, MAPK10), also known as stress-activated protein kinases (SAPKs).

3. p38 isoforms are p38α (MAPK14), p38β (MAPK11), p38γ (MAPK12 or ERK6) and p38δ (MAPK13 or SAPK4). Both JNK and p38 signaling pathways are responsive to stress stimuli, such as cytokines, ultraviolet irradiation, heat shock, and osmotic shock, and are involved in cell differentiation and apoptosis. p38α MAP Kinase (MAPK), also called RK or CSBP, is the mammalian orthologue of the yeast HOG kinase which participates in a signaling cascade controlling cellular responses to cytokines and stress. Similar to the SAPK/JNK pathway, p38 MAP kinase is activated by a variety of cellular stresses including osmotic shock, inflammatory cytokines, lipopolysaccharides (LPS), ultraviolet light and growth factors. p38 MAP kinase is activated by phosphorylation at Thr180 and Tyr182.

4. ERK5 (MAPK7), which has been found recently, is activated both by growth factors and by stress stimuli, and it participates in cell proliferation.

5. ERK3 (MAPK6) and ERK4 (MAPK4) are structurally related atypical MAPKs which possess an SEG (serine - glutamic acid - glycine) motif in the activation loop and display major differences only in the C-terminal extension.

6. ERK7/8 (MAPK15) are the most recently discovered members of the MAPK family and behave similar to ERK3/4.

Mitogen-activated protein kinase kinase forms a family of kinases which phosphorylates mitogen-activated protein kinase. They are also known as MAP2K and classified as EC 2.7.12.2. Seven genes exist. These encode MAP2K1 (MEK1), MAP2K2 (MEK2), MAP2K3 (MKK3), MAP2K4 (MKK4), MAP2K5 (MKK5), MAP2K6 (aka MKK6), MAP2K7 (MKK7). The activators of p38 (MKK3 and MKK4), JNK (MKK4), and ERK (MEK1 and MEK2) define independent MAP kinase signal transduction pathways.

Aurora kinases A (also known as Aurora, Aurora-2, AIK, AIR-1, AIRK1, AYK1, BTAK, Eg2, MmIAK1, ARK1 and STK15), B (also known as Aurora-1, AIM-1, AIK2, AIR-2, AIRK-2, ARK2, IAL-1 and STK12) and C (also known as AIK3) participate in several biological processes, including cytokinesis and dysregulated chromosome segregation. These important regulators of mitosis are over-expressed in diverse solid tumors. One member of this family of serine/threonine kinases, human Aurora A, has been proposed as a drug target in pancreatic cancer. The recent determination of the three-dimensional structure of Aurora A has shown that Aurora kinases exhibit unique conformations around the activation loop region. This property has boosted the search and development of inhibitors of Aurora kinases, which might also function as novel anti-oncogenic agents.

Glycogen synthase kinase 3 (GSK-3) is a serine/threonine protein kinase which in addition to the serine/threonine kinase activity has the unique ability to auto-phosphorylate on tyrosine residues. The phosphorylation of target proteins by GSK-3 usually inhibits their activity (as in the case of glycogen synthase and NFAT). GSK-3 is unusual among the kinases in that it usually requires a "priming kinase" to first phosphorylate a target protein and only then can GSK-3 additionally phosphorylate the target protein. In mammals GSK-3 is encoded by two known genes, GSK-3 alpha and beta. Aside from roles in pattern formation and cell proliferation during embryonic development, there is recent evidence for a role in tumor formation via regulation of cell division and apoptosis. Human glycogen synthase kinase-3 beta (GSK3β) is also associated with several pathophysiological conditions such as obesity, diabetes, Alzheimer's disease and bipolar disorder.

The Src family of proto-oncogenic tyrosine kinases transmit integrin-dependent signals central to cell movement and proliferation. The Src family includes nine members: Src, Lck, Hck, Fyn, Blk, Lyn, Fgr, Yes, and Yrk. These kinases have been instrumental to the modern understanding of cancer as a disease with disregulated cell growth and division. The c-Src proto-oncogene codes for the c-Src tyrosine kinase. Besides its kinase domain, c-Src is further comprised of an SH2 domain and an SH3 domain, which act as adaptor proteins for the formation of multi-enzyme complexes with the Src kinase domain. These domains are also involved in the auto-inhibition of the c-Src kinase domain. Mutations in this gene could be involved in the malignant progression of cancer cells. This protein specifically phosphorylates Tyr-504 residue on human leukocyte-specific protein tyrosine kinase (Lck), which acts as a negative regulatory site. It may also act on the Lyn and Fyn kinases.

Leukocyte-specific protein tyrosine kinase (Lck) is a protein that is found inside lymphocytes such as T-cells. Lck is a tyrosine kinase which phosphorylates tyrosine residues of certain proteins involved in the intracellular signaling pathways of lymphocytes. The N-terminal tail of Lck is myristoylated and palmitoylated, which tethers the protein to the plasma membrane of the cell. The protein furthermore contains an SH3 domain, an SH2 domain and in the C-terminal part the tyrosine kinase domain. The tyrosine phosphorylation cascade initiated by Lck culminates in the intracellular mobilization of calcium ($Ca^{2+}$) ions and activation of important signaling cascades within the lymphocyte. These include the Ras-MEK-ERK pathway, which goes on to activate certain transcription factors such as NFAT, NFκB, and AP-1 which then regulate the production of a plethora of gene products, most notably, cytokines such as Interleukin-2 that promote long-term proliferation and differentiation of the activated lymphocytes. Aberrant expression of Lck has been associated with thymic tumors, T-cell leukemia and colon cancers.

The catalytic activity of the Src family of tyrosine kinases is suppressed by phosphorylation on a tyrosine residue located near the C terminus (Tyr 527 in c-Src), which is catalyzed by C-terminal Src Kinase (Csk). Given the promiscuity of most tyrosine kinases, it is remarkable that the C-terminal tails of the Src family kinases are the only known targets of Csk. Interactions between Csk and c-Src, most likely representative for src kinases, position the C-terminal tail of c-Src at the edge of the active site of Csk. Csk cannot phosphorylate substrates that lack this docking mechanism because the conventional substrate binding site used by most tyrosine kinases to recognize substrates is destabilized in Csk by a deletion in the activation loop (Levinson, 2008).

The epidermal growth factor receptor (EGFR; ErbB-1; HER1 in humans) is the cell-surface receptor for members of the epidermal growth factor family (EGF-family) of extracellular protein ligands. The epidermal growth factor receptor is a member of the ErbB family of receptors, a subfamily of four closely related receptor tyrosine kinases: EGFR (ErbB-1), HER2/c-neu (ErbB-2), Her 3 (ErbB-3) and Her 4 (ErbB-4). Active EGFR occurs as a dimer. EGFR dimerization is induced by ligand binding to the extracellular receptor domain and stimulates its intrinsic intracellular protein-tyrosine kinase activity. As a result, autophosphorylation of several tyrosine residues in the C-terminal (intracellular) domain of EGFR occurs. This autophosphorylation elicits downstream activation and signaling by several other proteins that associate with the phosphorylated tyrosines through their own phosphotyrosine-binding SH2 domains. The kinase domain of EGFR can also cross-phosphorylate tyrosine residues of other receptors it is aggregated with, and can itself be activated in that manner. The EGFR signaling cascade activates several downstream signaling proteins which then initiate several signal transduction cascades, principally the MAPK, Akt and JNK pathways, leading to DNA synthesis and cell proliferation. Such pathways modulate phenotypes such as cell migration, adhesion, and proliferation. Mutations that lead to EGFR overexpression (known as upregulation) or overactivity have been associated with a number of cancers. Consequently, mutations of EGFR have been identified in several types of cancer, and it is the target of an expanding class of anticancer therapies.

The ABL1-protooncogene encodes a cytoplasmic and nuclear protein tyrosine kinase that has been implicated in processes of cell differentiation, cell division, cell adhesion and stress response. The activity of c-Abl protein is negatively regulated by its SH3 domain. A genetic deletion of the SH3 domain turns ABL1 into an oncogene. This genetic deletion, caused by the (9;22) gene translocation results in the head-to-tail fusion of the BCR (MIM:151410) and ABL1 genes present in many cases of chronic myelogeneous leukemia. The DNA-binding activity of the ubiquitously expressed ABL1 tyrosine kinase is regulated by CDC2-mediated phosphorylation, suggesting a cell cycle function for ABL1.

Discoidin domain receptor family, member 1, also known as DDR1 or CD167a (cluster of differentiation 167a), is a receptor tyrosine kinase (RTK) that is widely expressed in normal and transformed epithelial cells and is activated by various types of collagen. This protein belongs to a subfamily of tyrosine kinase receptors with a homology region similar to the *Dictyostelium discoideum* protein discoidin I in their extracellular domain. Its autophosphorylation is achieved by all collagens so far tested (type I to type VI). In situ studies and Northern-blot analysis showed that expression of this encoded protein is restricted to epithelial cells, particularly in the kidney, lung, gastrointestinal tract, and brain. In addition, this protein is significantly over-expressed in several human tumors from breast, ovarian, esophageal, and pediatric brain.

The kinases described above are preferred embodiments because all of them are involved in the development of diseases such as cancer for which at present not suitable cure is available or an improved treatment regimen is desired.

Using p38α, a kinase for which structural information was available, the present inventors demonstrated the applicability of the labeled kinase of the invention for screening purposes. Unexpectedly, the kinase could be prepared for labeling with a minimum of effort but also the labeled kinase exerted the desired properties, i.e. the introduced label proved suitable for the detection of conformational changes induced by binding of a specific inhibitor, in this case the known inhibitor BIRB-796 and several smaller BIRB-796 analogs.

In another preferred embodiment, the amino acid having a free thiol or amino group is cysteine, lysine, arginine or histidine.

Cysteine has a free thiol group, whereas lysine, arginine or histidine each possess at least one free amino group.

In another preferred embodiment, one or more solvent-exposed cysteines present outside the activation loop are deleted or replaced.

If more than one amino acid having a free thiol or amino group is present in a kinase of interest, specific labeling of the amino acid in the activation loop may not be possible. Therefore, as discussed above, amino acids having a free thiol or amino group should be deleted or replaced with another amino acid not having a free thiol or amino group if they are predicted or shown to be solvent-exposed. Cysteines which are naturally present in a kinase of interest and are solvent-exposed can be located outside the activation loop, in which case they should be deleted or replaced with another amino acid not having a free thiol group. This equally applies to amino acids having a free amino group which should then be replaced with an amino acid not having a reactive free amino group. In case that one or more amino acids having a free amino group is already present in the activation loop, amino acids having a free amino group and present in the activation loop in addition to the amino acid to be labeled, should be replaced or deleted, whichever of these mutations to the kinase does not inhibit its catalytic activity or interfere with its stability.

The term "solvent-exposed" refers to the position of an amino acid in the context of the three dimensional structure of the protein of which it is a part. Amino acids buried within the protein body are completely surrounded by other amino acids thus do not have any contact with the solvent. In contrast, solvent-exposed amino acids are partially or fully exposed to the surrounding solvent and are thus accessible to chemicals potentially able to modify them. This applies e.g. to thiol- or amino-reactive labels used in the present invention which can react with solvent-exposed amino acids having a free thiol- or amino-group.

The term "delete" refers to excision of an amino acid without replacing it with another amino acid whereas the term "replace" refers to the substitution of an amino acid with another amino acid. If an amino acid is replaced with another amino acid or deleted, the amino acid to be replaced or to be deleted is preferably chosen such that the amino acid deleted or replaced does not result in a kinase with inhibited catalytic activity and does not interfere with the stability of the resulting kinase.

In a more preferred embodiment, the kinase is p38α and a cysteine is introduced at position 172 of SEQ ID NO: 1 and preferably the cysteines at positions 119 and 162 of SEQ ID NO: 1 are replaced with another amino acid not having a free thiol group such as serine.

In general, amino acid replacements should be conservative. For cysteine, this means that it is preferably replaced with serine. In general, replacements of amino acids with different amino acids may be evaluated of whether they are conservative using the PAM250 Scoring matrix. The matrix is frequently used to score aligned peptide sequences to determine the similarity of those sequences (Pearson, 1990).

As described above, if not naturally present, an amino acid having a free thiol- or amino group has to be introduced into the activation loop of a kinase. In the case of p38α, structural studies were carried out using the available crystal structures for p38α in both the activated (DFG-in) and inactivated (DFG-out) state. P38α does not possess a cysteine in the activation loop. The above structural studies suggested that a replacement of alanine with a cysteine at

position 172, which is located in the activation loop, would not influence the catalytic activity or stability of the kinase.

The same studies revealed that two cysteines at positions 119 and 162 of SEQ ID NO: 1 are both solvent-exposed. To avoid potential interferences of the signals recorded for two additional cysteines not located in the activation loop, these two cysteines are preferably replaced with another amino acid, preferably with an amino acid similar in size and structure, such as serine.

If a kinase homologous to p38a is used, the position of the amino acid to be replaced with cysteine may correspond to position 172 in SEQ ID NO: 1. To determine which position in a kinase corresponds to position 172 in SEQ ID NO: 1, sequence alignments of SEQ ID NO: 1 with the used kinase can be effected, e.g. using publicly available programs such as CLUSTALW.

In another preferred embodiment, the thiol- or amino-reactive fluorophore is an environmentally sensitive di-substituted naphthalene compound of which one of the two substituents is a thiol- or amino-reactive moiety. The term "environmentally sensitive" denotes the sensitivity of the fluorophore to the conditions in its environment which is expressed in an alteration in its fluorescence emission at one or more wavelengths or in its complete emission spectrum. Conditions causing such alteration are e.g. changes in the polarity or conformational changes in the activation loop.

The above types of fluorophores typically exhibit changes in both intensity and a shift in the emission wavelength depending on the polarity of the surrounding environment. Examples of this class of fluorophores include 6-acryloyl-2-dimethylaminonaphthalene (Acrylodan), 6-bromoacetyl-2-dimethylamino-naphthalenebadan (Badan), 2-(4'-(iodoacetamido)anilino)naphthalene-6-sulfonic acid, sodium salt (IAANS), 2-(4'-maleimidylanilino)naphthalene-6-sulfonic acid, sodium salt (MIANS), 5-((((2-iodoacetyl)amino)ethyl)amino)naphthalene-1-sulfonic acid (1,5-IAEDANS) and 5-dimethylaminonaphthalene-1-sulfonyl aziridine (dansyl aziridine) or a derivative thereof.

Other fluorophores which may be used due to their environmental sensitivity are coumarin-based compounds, benzoxadiazole-based compounds, dapoxyl-based compounds, biocytin-based compounds, fluorescein, sulfonated rhodamine-based compounds such as AlexaFluor dyes (Molecular Probes), Atto fluorophores (Atto Technology) or Lucifer Yellow. Coumarin-based fluorophores are moderately sensitive to environment and 7-diethylamino-3-(4'-maleimidylphenyl)-4-methylcoumarin (CPM) is an example. Benzoxadiazole fluorophores are also commonly used for forming protein-fluorophore conjugates and have a strong environmental dependence with 7-fluorobenz-2-oxa-1,3-diazole-4-sulfonamide (ABD-F) and *N*-((2-(iodoacetoxy)ethyl)-*N*-methyl)amino-7-nitrobenz-2-oxa-1,3-diazole ester (IANBD) as examples. PyMPO maleimide (for thiols) or succinimide ester (for amines) and various other dapoxyl dyes have good absorptivity and exceptionally high environmental sensitivity. Examples are 1-(2-maleimidylethyl)-4-(5-(4-methoxyphenyl)oxazol-2-yl)pyridinium methanesulfonate (PyMPO-maleimide), 1-(3-(succinimidyloxycarbonyl)benzyl)-4-(5-(4-methoxyphenyl) oxazol-2-yl) pyridinium bromide (PyMPO-succinimidyl ester) and Dapoxyl (2-bromoacetamidoethyl) sulphonamide. However, due to their longer more flexible structures, these probes may effect activation loop movement depending on the labeling site chosen. As demonstrated in the appended examples, pyrene could be used as a label but did not prove to be preferable. The applicability of the above substances depends on the individual kinase and the position of the amino acid to be labeled so that they can in principle be applied as labels as well, even if in some cases they may cause a reduced sensitivity in the methods of the invention. Matching the above substances with a suitable kinase can be performed by the skilled artisan using routine procedures in combination with the teachings of this invention. In general, any fluorophore can be used as long as it does not inhibit the catalytic activity or interfere with the stability of the kinase. This means that the fluorophore is preferably not bulky or extended.

In a further preferred embodiment, the thiol-reactive spin-label is a nitroxide radical.

The dominant method for site-specifically labeling protein sequences with a spin-label is the reaction between methanethiosulfonate spin label and cysteine, to give the spin-labeled cysteine side chain, CYS-SL:

MeS(O)2SSR + R'SH ---> R'SSR + MeS(O)2SH

where R is the nitroxide group and R'SH is a protein with a cysteine sulfhydryl, and R'SSR is the spin-labeled protein. The cysteines for labeling are placed in the desired sequence position either through solid-phase techniques or through standard recombinant DNA techniques.

The present invention furthermore relates to a method of screening for kinase inhibitors comprising (a) providing a fluorescently or spin-labeled or isotope-labeled kinase according to the invention; (b) contacting said fluorescently or spin-labeled or isotope-labeled kinase with a candidate inhibitor; (c) recording the fluorescence emission signal at one or more wavelengths or a spectrum of said fluorescently labeled kinase of step (a) and step (b) upon excitation; or (c)' recording the electron paramagnetic resonance (EPR) or nuclear magnetic resonance (NMR) spectra of said spin-labeled or isotope-labeled kinase of step (a) and step (b); and (d) comparing the fluorescence emission signal at one or more wavelengths or the spectra recorded in step (c) or the EPR or NMR spectra recorded in step (c)'; wherein a difference in the fluorescence intensity at at least one wavelength, preferably at the emission maximum and/or a shift in the fluorescence emission wavelength in the spectra of said fluorescently labeled kinase obtained in step (c), or an alteration in the EPR or NMR spectra of said spin-labeled or isotope-labeled kinase obtained in step (c)' indicates that

the candidate inhibitor is a kinase inhibitor.

Kinase inhibitors are substances capable of inhibiting the activity of kinases. They can more specifically inhibit the action of a single kinase, e.g. if they are allosteric inhibitors (Type III) or those binding to the allosteric site adjacent to the ATP-binding site and reaching into the ATP-binding pocket (Type II). Alternatively, an inhibitor can inhibit the action of a number of protein kinases, which is particularly the case if it binds exclusively to the ATP-binding pocket (Type I), which is very conserved among protein kinases.

A candidate inhibitor may belong to different classes of compounds such as small organic or inorganic molecules, proteins or peptides, nucleic acids such as DNA or RNA. Such compounds can be present in molecule libraries or designed from scratch. Small molecules according to the present invention comprise molecules with a molecular weight of up to 2000 Da, preferably up to 1500 Da, more preferably up to 1000 Da and most preferably up to 500 Da.

Recording the fluorescence emission signal at one or more wavelengths or a spectrum is usually accomplished using a fluorescence spectrometer or fluorimeter. Fluorescence spectroscopy or fluorimetry or spectrofluorimetry is a type of electromagnetic spectroscopy which analyzes fluorescence, or other emitted light, from a sample. It involves using a beam of light, usually ultraviolet light, that excites the electrons in certain molecules and causes them to emit light of a lower energy upon relaxation, typically, but not necessarily, visible light.

Two general types of instruments exist which can both be employed in the method of the invention: Filter fluorimeters use filters to isolate the incident light and fluorescent light, whereas spectrofluorimeters use diffraction grating monochromators to isolate the incident light and fluorescent light. Both types utilize the following scheme: The light from an excitation source passes through a filter or monochromator and strikes the sample. A proportion of the incident light is absorbed by the sample, and some of the molecules in the sample fluoresce. The fluorescent light is emitted in all directions. Some of this fluorescent light passes through a second filter or monochromator and reaches a detector, which is usually placed at 90° to the incident light beam to minimize the risk of transmitted or reflected incident light reaching the detector. Various light sources may be used as excitation sources, including lasers, photodiodes, and lamps; xenon and mercury vapor lamps in particular. The detector can either be single-channeled or multi-channeled. The single-channeled detector can only detect the intensity of one wavelength at a time, while the multi-channeled detects the intensity at all wavelengths simultaneously, making the emission monochromator or filter unnecessary. The different types of detectors have both advantages and disadvantages. The most versatile fluorimeters with dual monochromators and a continuous excitation light source can record both an excitation spectrum and a fluorescence spectrum. When measuring fluorescence spectra, the wavelength of the excitation light is kept constant, preferably at a wavelength of high absorption, and the emission monochromator scans the spectrum. For measuring excitation spectra, the wavelength passing though the emission filter or monochromator is kept constant and the excitation monochromator is scanning. The excitation spectrum generally is identical to the absorption spectrum as the fluorescence intensity is proportional to the absorption (for reviews see Rendell, 1987; Sharma and Schulman, 1999; Gauglitz and Vo-Dinh, 2003; Lakowicz, 1999).

Nuclear magnetic resonance (NMR) is a physical phenomenon based upon the quantum mechanical magnetic properties of the nucleus of an atom. All nuclei that contain odd numbers of protons or neutrons have an intrinsic magnetic moment and angular momentum. The most commonly measured nuclei are hydrogen ($^{1}$H) (the most receptive isotope at natural abundance) and carbon ($^{13}$C), although nuclei from isotopes of many other elements (e.g.$^{113}$Cd, $^{15}$N, $^{14}$N $^{19}$F, $^{31}$P, $^{17}$O, $^{29}$Si, $^{10}$B, $^{11}$B, $^{23}$Na, $^{35}$Cl, $^{195}$Pt) can also be observed. NMR resonant frequencies for a particular substance are directly proportional to the strength of the applied magnetic field, in accordance with the equation for the Larmor precession frequency. NMR measures magnetic nuclei by aligning them with an applied constant magnetic field and perturbing this alignment using an alternating magnetic field, those fields being orthogonal. The resulting response to the perturbing magnetic field is the phenomenon that is exploited in NMR spectroscopy and magnetic resonance imaging, which use very powerful applied magnetic fields in order to achieve high spectral resolution, details of which are described by the chemical shift and the Zeeman Effect.

In the present invention, a suitable amino acid in the activation loop can be labeled with an isotope or thiol/amine-reactive small molecule containing enriched isotopes. In this case, the only signal comes from the enriched molecule on the activation loop, which is sensitive to protein conformation depending on the labeling site chosen.

Preferred isotopes are $^{13}$C, $^{15}$N, etc. which can be measured as 1D or 2D NMR spectra. Changes in protein conformation, e.g. due to the binding of an inhibitor will result in a shift of the NMR chemical shift(s) corresponding to the label.

Electron paramagnetic resonance (EPR) or electron spin resonance (ESR) spectroscopy, as has been briefly described above, is a technique for studying chemical species that have one or more unpaired electrons, such as organic and inorganic free radicals or inorganic complexes possessing a transition metal ion. The basic physical concepts of EPR are analogous to those of nuclear magnetic resonance (NMR), but it is electron spins that are excited instead of spins of atomic nuclei. Because most stable molecules have all their electrons paired, the EPR technique is less widely used than NMR. However, this limitation to paramagnetic species also means that the EPR technique is one of great specificity, since ordinary chemical solvents and matrices do not give rise to EPR spectra.

The EPR technique utilizes spin-labels. In this case, the kinase, to be examined is expressed in bacteria or other suitable

host cells in the presence of an isotope such as $^{13}C$ and $^{15}N$ resulting in the incorporation of these isotopes throughout the entire protein as it is expressed. After purification of the isotope enriched protein, a spin label is attached to the activation loop as described above. In this case, 2D NMR spectra of the isotopes in the protein are recorded. As the activation loop and spin label change conformation, the spin label will induce a change in some of the protein signals coming from the incorporated isotopes which come into closer contact with the activation loop or spin label as inhibitors bind. Peaks would become broader as the spin label approaches.

Different EPR spectra or fluorescence emission signals at one or more wavelengths, preferably at the emission maximum, or different fluorescence emission spectra obtained in step (c) or (c)' indicate a conformational change in the kinase caused by binding of the candidate compound. This is due to the fact that binding of a compound to the allosteric site adjacent to the ATP-binding pocket, and in some cases to the ATP-binding pocket itself, results in a perturbation of the DFG motif, a conformational change in the activation loop, a polarity change and/or a change in the interaction of free electrons in an attached spin-label with the nuclei of adjacent atoms. Upon comparison of the EPR or NMR spectra or the fluorescence emission, the present method reveals whether a candidate compound qualifies as a suitable kinase inhibitor, e.g. not only a high-affinity inhibitor but also one which specifically inhibits the activity of one kinase. The data recorded for the kinase without a candidate inhibitor and those recorded for the kinase having been contacted with said candidate inhibitor are compared. In case of fluorescence emission signal either the signal at one or more specific wavelengths can be recorded and compared enabling for a detection of a change in the intensity of the signal at the particular wavelength(s). Alternatively, a complete spectrum can be recorded and compared enabling also for the observation of changes in the maximum emission wavelength.

Preferably, said method is effected in high-throughput format. High-throughput assays, independently of being biochemical, cellular or other assays, generally may be performed in wells of microtiter plates, wherein each plate may contain 96, 384 or 1536 wells. Handling of the plates, including incubation at temperatures other than ambient temperature, and bringing into contact with test compounds, in this case putative inhibitors, with the assay mixture is preferably effected by one or more computer-controlled robotic systems including pipetting devices. In case large libraries of test compounds are to be screened and/or screening is to be effected within short time, mixtures of, for example 10, 20, 30, 40, 50 or 100 test compounds may be added to each well. In case a well exhibits inhibitory activity, said mixture of test inhibitors may be de-convoluted to identify the one or more test inhibitors in said mixture giving rise to said activity.

Alternatively, only one test inhibitor may be added to a well, wherein each test inhibitor is applied in different concentrations. For example, the test inhibitor may be tested in two, three or four wells in different concentrations. In this initial screening, the concentrations may cover a broad range, e.g. from 10 nM to 10 μM. The initial screening serves to find hits, i.e. test inhibitors exerting inhibiting activity at at least one concentration, preferably two, more preferably all concentrations applied, wherein the hit is more promising if the concentration at which an inhibitory activity can be detected is in the lower range. This alternative serves as one preferred embodiment in accordance with the invention.

Test inhibitors considered as a hit can then be further examined using an even wider range of inhibitor concentrations, e.g. 10 nM to 20 μM. The method applied for these measurements is described in the following.

The present invention furthermore relates to a method of determining the kinetics of ligand binding and/or of association or dissociation of a kinase inhibitor comprising (a) contacting a fluorescently labeled kinase according to the invention with different concentrations of an inhibitor; or (a)' contacting a fluorescently labeled kinase according to the invention bound to an inhibitor with different concentrations of unlabelled kinase; (b) recording the fluorescence emission signal at one or more wavelengths or a spectrum of said fluorescently labeled kinase for each concentration of inhibitor and/or unlabeled kinase upon excitation; (c) determining the rate constant for each concentration from the fluorescence emission signals at one or more wavelengths or the spectra recorded in step (b) or (c1) determining the $K_d$ from the fluorescence emission signal at one or more wavelengths or the spectra recorded in step (b) for each concentration of inhibitor; or (c2) determining the $K_a$ or inverse $K_d$ from the fluorescence emission signal at one or more wavelengths or the spectra recorded in step (b) for each concentration of unlabelled kinase; (d) directly determining the $k_{on}$ and/or extrapolating the $k_{off}$ from the rate constants determined in step (c) from the signals or spectra for the different concentrations of inhibitor obtained in step (b); or (d)' directly determining the $k_{off}$ and/or extrapolating the $k_{on}$ from the rate constants determined in step (c) from the signals or spectra for the different concentrations of unlabelled kinase obtained in step (b); and optionally (e) calculating the $K_d$ and/or $K_a$ from $k_{on}$ and $k_{off}$.obtained in step (d) or (d)'.

By contacting a labeled kinase with different concentrations of an inhibitor, and subsequently determining the fluorescence emission for each concentration applied, the binding affinity of an inhibitor can be measured. For each concentration, the ratio of bound and unbound inhibitor will be different, reflecting the increasing concentration of inhibitor but also the specific binding affinity of said inhibitor to said kinase.

The opposite approach can be followed by titrating a labeled kinase containing a bound inhibitor with unlabeled kinase with no inhibitor bound.

In chemical kinetics, a rate constant k quantifies the speed of a chemical reaction. For a chemical reaction where substance A and B are reacting to produce C, the reaction rate has the form:

$$\frac{d[C]}{dt} = k(T)[A]^m[B]^n$$

Wherein k(T) is the reaction rate constant that depends on temperature.
[A] and [B] are the concentrations of substances A and B, respectively, in moles per volume of solution assuming the reaction is taking place throughout the volume of the solution.

The exponents m and n are the orders and depend on the reaction mechanism. They can be determined experimentally.
A single-step reaction can also be described as:

$$\frac{d[C]}{dt} = Ae^{\frac{-E_a}{RT}}[A]^m[B]^n$$

$E_a$ is the activation energy and R is the Gas constant. Since at temperature *T* the molecules have energies according to a Boltzmann distribution, one can expect the proportion of collisions with energy greater than $E_a$ to vary with $e^{-Ea/RT}$.
*A* is the pre-exponential factor or frequency factor.

$k_{on}$ and $k_{off}$ are constants that describe non-covalent equilibrium binding. When a ligand interacts with a receptor, or when a substrate interacts with an enzyme, the binding follows the law of mass action.

$$R + L \underset{k_{off}}{\overset{k_{on}}{\rightleftharpoons}} RL$$

In this equation R is the concentration of free receptor, L is the concentration of free ligand, and RL is the concentration of receptor-ligand complex. In the case of enzyme kinetics, R is the enzyme, or in this case a protein kinase, and L is the substrate, or in this case a candidate or known inhibitor. The association rate constant $k_{on}$ is expressed in units of $M^{-1}sec^{-1}$. The rate of RL formation equals R x L x $k_{on}$. The dissociation rate constant $k_{off}$ is expressed in units of $sec^{-1}$. The rate of RL dissociation equals RL x $k_{off}$. At equilibrium, the backward (dissociation) reaction equals the forward (association) reaction. Binding studies measure specific binding, which is a measure of RL. Enzyme kinetic assays assess enzyme velocity, which is proportional to RL, the concentration of enzyme-substrate complexes.

$$RL = R \cdot L \cdot \frac{k_{on}}{k_{off}}$$

The equilibrium dissociation constant, Kd is expressed in molar units and defined to equal $k_{off}/k_{on}$ to arrive at

$$RL = R \cdot L \cdot \frac{k_{on}}{k_{off}} = \frac{R \cdot L}{K_d}$$

The dissociation constant ($K_d$) corresponds to the concentration of ligand (L) at which the binding site on a particular protein is half occupied, i.e. the concentration of ligand, at which the concentration of protein with ligand bound (RL), equals the concentration of protein with no ligand bound (R). The smaller the dissociation constant, the more tightly bound the ligand is, or the higher the affinity between ligand and protein.
Accordingly, the association constant $K_a$, also called inverse Kd, is defined as $1/k_d$. The dissociation constant for a particular ligand-protein interaction can change significantly with solution conditions (e.g. temperature, pH and salt

concentration).

Depending on which sequence of steps is followed in the above method of the invention, the $K_d$ or $K_a$ can be measured directly or indirectly.

For directly measuring the $K_d$or the $K_a$, respectively, step (c1) or (c2) which is the last step for this type of measurement follows step (b). This type of measurement is called endpoint measurement and also illustrated in the appended examples. Unlike for indirectly determining $K_d$ or $K_a$ through calculation using rate constants, the final fluorescence emission at equilibrium is measured rather than the fluorescence change over time. These measurements can be used to generate a binding curve using different inhibitor concentrations (for determining $K_d$) or concentrations of unlabelled kinase (for determining $K_a$). From these curves, $K_d$ or $K_a$ can be obtained directly.

For indirectly obtaining $K_d$ or $K_a$, the rate constants from the fluorescence emission signal at one or more wavelengths or the spectra recorded in step (b) have to be determined for each concentration as done in step (c). Depending the type of titration, i.e. titration of labeled kinase, with inhibitor or titration of labeled kinase bound to inhibitor with unlabeled kinase, either $k_{on}$ or $k_{off}$ can be determined directly from the measured rate constants. For determining $k_{on}$, step (d) is applied which also enables for extrapolation of $k_{off}$. Accordingly, step (d)' is applied for directly determining $k_{off}$ which in turn enables for extrapolation of $k_{on}$. From $k_{on}$ and/or $k_{off}$ obtained in steps (d) or (d)', the $K_d$ and/or $K_a$ can be calculated according to the equations discussed above.

The above method may also be applied in high-throughput screens. If a compound exerting inhibitory activity on a kinase has been identified, e.g. using the method of screening for kinase inhibitors of the invention, the present method can be used to further characterize said inhibitor. For example, the high-throughput format can be used to determine the Ka or Kd from the fluorescence emission signal at one or more wavelengths for multiple different concentrations of inhibitors (variant (a)) or, unlabelled kinase (variant (b)). Concentration ranges to be tested reach for example from 10 nM to 20 $\mu$M such that repeating series of 1, 2 and 5 (i.e. 10, 20, 50, 100, 200, 500 nM, etc.) between the concentrations assessed.

In a different embodiment, the present invention relates to a method of determining the dissociation or association of a kinase inhibitor comprising (a) contacting a spin-labeled or isotope-labeled kinase according to the invention with different concentrations of an inhibitor; or (a)' contacting a spin-labeled or isotope-labeled kinase according to the invention bound to an inhibitor with different concentrations of unlabelled kinase; (b) recording the EPR or NMR spectrum of said spin-labeled or isotope-labeled kinase for each concentration of inhibitor and/or unlabelled kinase; and (c) determining the $K_d$ from the EPR or NMR spectra recorded in step (b) for the different concentrations of inhibitor; or (c)' determining the $K_a$ from the EPR or NMR spectra recorded in step (b) for the different concentrations of unlabeled kinase.

Similar to the method disclosed further above relating to determining the kinetic constants using fluorescently labeled kinase, the present method allows for the direct determination of the association or dissociation constants for the reaction a kinase and an inhibitor. Unlike for fluorescently labeled kinases, the instrumental limitations and time required to collect NMR and EPR measurements are, in most cases, not compatible with the fast time scale of inhibitor binding and do not allow the direct determination of $k_{on}$ or $k_{off}$. Determinations for compounds which require several hours to bind to the kinase may also be possible.

The methods of the invention relating to determining kinetic data can also be applied to a high-throughput format. For example, a potential inhibitor identified with the screening method of the invention described above can be further characterized in that different concentrations of said inhibitor are applied to the kinase to determine the Kd. Suitable but not limiting concentration ranges for the inhibitor are between 10 nM and 20 $\mu$M.

More focused concentration ranges applied in the high-throughput format may serve to obtain more sensitive Kd measurements, e.g. with the cuvette approach and real-time kinetics measurements as done in the appended examples, by determining $k_{on}$ and $k_{off}$.

The present invention furthermore relates to a method of generating mutated kinases suitable for the screening of kinase inhibitors comprising (a) replacing solvent exposed amino acids having a free thiol or amino group, if any, present in a kinase of interest outside the activation loop or amino acids having a free thiol or amino group at an unsuitable position within the activation loop with an amino acid not having a free thiol or amino group; (b) mutating an amino acid in the activation loop of said kinase of interest to an amino acid having a free thiol or amino group if no amino acid having a free thiol or amino group is present in the activation loop; (c) labeling the kinase of interest with a thiol- or amino-reactive fluorophore sensitive to polarity changes in its environment, a thiol-reactive spin label, an isotope or an isotope-enriched thiol- or amino-reactive label such that said fluorophore, spin label, isotope or isotope-enriched label does not inhibit the catalytic activity and/or does not interfere with the stability of the kinase; (d) contacting the kinase obtained in step (c) with a known inhibitor of said kinase; and (e) recording the fluorescence emission signal at one or more wavelengths or a spectrum of said fluorescently labeled kinase of step (c) and (d) upon excitation or (e)' recording the EPR or NMR spectra of said spin-labeled kinase of step (c) and (d); and (f) comparing the fluorescence emission signal at one or more wavelengths or the spectrum recorded in step (e) or the EPR or NMR spectra recorded in step (e)'; wherein a difference in the fluorescence intensity at at least one wavelength, preferably the emission maximum and/or a shift in the fluorescence emission wavelength in the spectra of said fluorescently labeled kinase obtained in step (e),

or an alteration in the EPR or NMR spectra of said spin-labeled or isotope-labeled kinase obtained in step (e)' indicates that the kinase is suitable for the screening for kinase inhibitors.

Adapted to a high-throughput format, multiple kinases or differently labeled variations of the same kinase can be screened.

The term "unsuitable position" in accordance with the present invention denotes a position in the activation loop which was shown to be not suitable for an amino acid labeled according to the invention. This can be due to a decreased sensitivity of the label to changes in its environment or due to predictions based on structural considerations that said position would result in a kinase with a label with decreased sensitivity. The term also encompasses amino acids positioned at a potentially suitable position, wherein a different position is deemed more appropriate. As soon as the number of amino acids having a free thiol or amino group in the activation loop exceeds one, amino acids deemed as unsuitable should be mutated.

Mutating an amino acid includes replacing or deleting said amino acid with another amino acid, provided that said mutation does not result in an inhibited catalytic activity or an interference with the stability of the resulting kinase. Step (b) is carried out if no amino acid having a free thiol or amino group is present in the activation loop of said kinase of interest. The amino acid which is inserted or which replaces another amino acid has to have a free thiol or amino group in order to be labeled.

In a preferred embodiment of the methods of the present invention, the kinase inhibitor binds either exclusively to the allosteric site adjacent to the ATP binding site of the kinase or extends from the allosteric site into the ATP site. These types of inhibitors are also called Type III or Type II inhibitors, respectively. They bind to kinases with higher specificity as compared to Type I inhibitors which bind to the ATP-pocket of the kinase, which is highly conserved in structure among all kinases.

As demonstrated in the examples, the present invention provides means to differentiate between ATP-competitive and non-ATP-competitive inhibitor, enabling for a rapid election of specific inhibitors. The invention is designed to detect the movement of the activation loop of the kinase and is therefore sensitive to all Type II and Type III inhibitors. Although certain Type I inhibitors are either not detected at all or are weakly detected only at high concentrations, some of these inhibitors have induced a robust fluorescence change. Only measurement of the fluorescence change over time (i.e. not an endpoint measurement) can allow Type I inhibitors to be distinguished. As presented in one of the examples below, detected ATP-competitive inhibitors produce an instantaneous fluorescence change (typically < 5-10 sec) while Type II and Type III inhibitors bind much slower (seconds to several minutes).

In another preferred embodiment of the kinase or the methods of the present invention, the kinase is labeled at a cysteine naturally present or introduced into the activation loop.

The abundance of cysteines in proteins is usually very low, so that a kinase of the invention can be prepared in a straightforward manner by replacing an amino acid in the activation loop with cysteine and optionally replacing solvent-exposed cysteines with other amino acids. Amino acids containing reactive amines, such as histidine, arginine or lysine or derivatives thereof, are much more abundant and are readily found at the protein surface where they are in contact with the surrounding solvent. Thus, it is preferable to use thiol-reactive labels which can specifically react with an introduced cysteine.

In a more preferred embodiment, the method of screening for kinase inhibitors or the method of generating mutated kinases further comprises step (c1) measuring a fluorescence intensity ratio of two wavelengths recorded in step (c) and obtaining the ratio of the normalized intensity change to the average intensity change ($\Delta I_{std}$). Additionally or alternatively, the maximum standard intensity change ($\Delta R_{max}$) between a kinase labeled according to the invention with inhibitor bound and one without inhibitor may be assessed. A candidate compound is considered a kinase inhibitor or the fluorescent- labeled kinase is considered suitable for the screening for kinase inhibitors if ($\Delta I_{std}$) is > 0.25, and/or ($\Delta R_{max}$) is > 0.75 and the Z-factor is > 0.5. This embodiment relates to the extension of the methods of the present invention to high-throughput scale as described above.

$\Delta I_{std}$ is the ratio of normalized intensity change to average intensity of the fluorescence emission. According to de Lorimier et al. (2002), $\Delta I_{std}$ is one of the most important criteria for characterizing a fluorescent protein conjugate as suitable for sensitive fluorescence spectroscopy. Ideally, the $\Delta I_{std}$ should have a value > 0.25 and is calculated by:

$$\Delta I_{std} = \left| \frac{2(I_1(\lambda_{std}) - I_2(\lambda_{std}))}{I_1(\lambda_{std}) + I_2(\lambda_{std})} \right|$$

where $\lambda_{std} = (\lambda_{max}$, unbound $+ \lambda_{max,\ saturated})/2$ and $I_1$, $I_2$ are the fluorescence intensities at $\lambda_{std}$ of each spectrum respectively.

$\Delta R_{max}$ is the maximum standard intensity change of the fluorescence emission between saturated and unsaturated kinase (REF). According to (de Lorimier et al., 2002), $\Delta R_{max}$ is another important criteria for characterizing a fluorescent protein conjugate as suitable for sensitive fluorescence spectroscopy. Ideally, the $\Delta R_{max}$ should have a value > 1.25 and is calculated by:

$$\Delta R = \left| \frac{{}^{0}A_1}{{}^{0}A_2} - \frac{{}^{\infty}A_1}{{}^{\infty}A_2} \right|$$

where ${}^{\circ}A_1$, ${}^{\circ}A_2$ are the areas in the absence of ligand, and ${}^{\infty}A_1$, ${}^{\infty}A_2$ are the areas in the presence of saturating ligand. A computer program can be used to enumerate $\Delta R$ for all possible pairs of wavelength bands in the two spectra, to identify the optimal sensing condition, defined as the maximum value of $\Delta R$.

The Z-factor is a statistical measure of the quality or power of a high-throughput screening (HTS) assay. In an HTS campaign, large numbers of single measurements of unknown samples are compared to well established positive and negative control samples to determine which, if any, of the single measurements are significantly different from the negative control. Prior to starting a large screening campaign, much work is done to assess the quality of an assay on a smaller scale, and predict if the assay would be useful in a high-throughput setting. The Z-factor predicts if useful data could be expected if the assay were scaled up to millions of samples. The Z-factor is calculated by:

$$Zfactor = 1 - \frac{3 \times (\sigma_p + \sigma_n)}{|\mu_p - \mu_n|}$$

wherein both the mean ($\mu$) and standard deviation ($\sigma$) of both the positive (p) and negative (n) controls ($\mu_p$,$\sigma_p$,$\mu_n$,$\sigma_n$, respectively) are taken into account.

The measurement of $\Delta I_{std}$ and $\Delta R_{max}$ as well as the determination of the Z-factor may prove useful in determining whether the label chosen is suitable in the screening for inhibitors. De Lorimier discusses that the measured kinetics and Kd obtained with a fluorescent tagged protein will depend on the protein, the ligand and the fluorophore used. Therefore, the same inhibitor binding to the same kinase could give different Kd values depending on the label used. The determination of the above values might indicate whether the label chosen is appropriate or whether a different label should be used.

In a further preferred embodiment, the fluorophore or spin-label is not located at or adjacent to phosphorylation sites known or predicted to exist in the labeled kinase. This ensures that the labeling does not interfere with the dynamics of the activation loop or the normal activity and regulation of the kinase which is largely affected by phosphorylation and dephosphorylation.

In another preferred embodiment, said candidate amino acid in the activation loop is identified based on structural and/or sequence data available for said kinase.

For some kinases, structural data, e.g. in the form of crystal or NMR structures is available, wherein the kinase is captured in the activated and/or inactivated state. If such data is available for a kinase, this facilitates the choice of the amino acid position in the activation loop to be replaced for labeling purposes. The actual choice is based on the distance of the position from the allosteric site of the kinase as well as on contacts of the amino acid in said position with other amino acids. If said contacts are deemed essential for the catalytic activity or stability of the kinase, the position is in most cases not suitable for replacement. Additionally, the choice is based on the distance which a particular amino acid will move as the protein changes conformation such that greater distances increase the chance that an environmental change will be detected. However, although distance moved is an indicator of whether a particular position may be useful for labeling, it is the actual change in environment which will correlate directly with the observed changes detected by the attached label.

In a preferred embodiment, the methods of the present invention relating to screening for inhibitors, determining kinetic parameters such as association and dissociation and generating a mutated kinase are combined to obtain a straightforward methodology to obtain specific inhibitors for different kinases. In this regard, any preferred embodiment of a method of the invention may be combined with embodiments of other methods of the invention. In a more preferred embodiment of this aspect, an initial screen is carried out using the method of high-throughput screening for kinase inhibitors, followed by a screen using a wide range of concentrations of inhibitors as described above with the method of the invention for determining the kinetics of ligand binding and/or association or dissociation. The latter step is carried

out, inter alia, to get an indication of the Kd and/or Ka value. This step is again repeated by carrying out measurements with a more focused concentration range for more precise measurements of the Kd or Ka. These measurements may be carried out either as a titration series with the cuvette approach and/or real-time kinetic measurements in cuvettes ($k_{on}$ and $k_{off}$) to further characterize each inhibitor. Optionally, this sequence of methods is transferred to other kinases or the same kinase labeled differently. This embodiment is designed to enable for high-throughput screening to screen for and characterize a high number of inhibitors in multiple kinases or differently labeled variations of the same kinase.

In another preferred embodiment of the method for screening of kinase inhibitors, the method further comprises (subsequently) optimizing the pharmacological properties of a candidate compound identified as inhibitor of said kinase.

Methods for the optimization of the pharmacological properties of compounds identified in screens, generally referred to as lead compounds, are known in the art and comprise a method of modifying a compound identified as a lead compound to achieve: (a) modified site of action, spectrum of activity, organ specificity, and/or (b) improved potency, and/or (c) decreased toxicity (improved therapeutic index), and/or (d) decreased side effects, and/or (e) modified onset of therapeutic action, duration of effect, and/or (f) modified pharmacokinetic parameters (absorption, distribution, metabolism and excretion), and/or (g) modified physico-chemical parameters (solubility, hygroscopicity, color, taste, odor, stability, state), and/or (h) improved general specificity, organ/tissue specificity, and/or (i) optimized application form and route by a. esterification of carboxyl groups, or b. esterification of hydroxyl groups with carboxylic acids, or c. esterification of hydroxyl groups to, e.g. phosphates, pyrophosphates or sulfates or hemi-succinates, or d. formation of pharmaceutically acceptable salts, or e. formation of pharmaceutically acceptable complexes, or f. synthesis of pharmacologically active polymers, or g. introduction of hydrophilic moieties, or h. introduction/exchange of substituents on aromates or side chains, change of substituent pattern, or i. modification by introduction of isosteric or bioisosteric moieties, or j. synthesis of homologous compounds, or k. introduction of branched side chains, or I. conversion of alkyl substituents to cyclic analogues, or m. derivatization of hydroxyl group to ketales, acetales, or n. N-acetylation to amides, phenylcarbamates, or o. synthesis of Mannich bases, imines, or p. transformation of ketones or aldehydes to Schiffs bases, oximes, acetales, ketales, enolesters, oxazolidines, thiazolidines or combinations thereof.

The various steps recited above are generally known in the art. They include or rely on quantitative structure-action relationship (QSAR) analyses (Kubinyi, "Hausch-Analysis and Related Approaches", VCH Verlag, Weinheim, 1992), combinatorial biochemistry, classical chemistry and others (see, for example, Holzgrabe and Bechtold, Deutsche Apotheker Zeitung 140(8), 813-823, 2000).

The figures show

**Figure 1**

p38α has been crystallized in its active (DFG-in) and inactive state (DFG-out) (A). The pyrazolo-urea compound BIRB-796 is a Type II inhibitor which extends between the ATP and allosteric binding sites of p38α.. Attachment of acrylodan (modeled here as a tryptophan) to a selected Cys (B) mutation in the activation loop should detect conformational changes that result from the binding of Type II and III inhibitors (C). Upon binding, BIRB-796 alters the conformation of the activation loop (red) (D).

**Figure 2**

The binding of allosteric inhibitors results in a large decrease in acrylodan emission at 468 nm and a characteristic red-shift to 514 nm in the ligand-bound (inactive) state.

**Figure 3**

Real-time and endpoint fluorescence measurements using ac-p38α labeled on the activation loop. Acrylodan emission at 468 nm decreases in a dose-dependent manner upon binding of BIRB-796 (A). Fluorescence traces follow first-order decay kinetics and can be plotted to determine $k_{on}$ and $k_{off}$ for BIRB-796 (B). Endpoint equilibrium measurements can also be made to obtain the Kd of binding. Raw fluorescence data (R = 514 nm / 468 nm) were plotted to show the saturation of the inactive state (C). The Kd was determined by using a logarithmic scale plotted against R and fractional occupancy (D).

**Figure 4**

Titration of sorafenib (inhibitor for b-Raf and p38α), lapatinib (inhibitor for EGFR and HER2) (inhibitor for Abl, c-Kit) and imatinib with ac-p38α. Fluorescent-tagged p38α was incubated with various inhibitor concentrations overnight prior to making endpoint measurements (left). Imatinib and lapatinib did not bind to ac-p38α (as expected) in the concentration range examined, while sorafenib bound tightly with a Kd ~56 nM. The structures of each inhibitor are also shown (right).

**Figure 5**

Dissociation of BIRB-796 and MG001 from ac-p38α and direct measurements of koff. BIRB-796 or MG001 were mixed with ac-p38α (0.1 μM) in a 1:1 ratio. After sufficient incubation time, 1 μM unlabeled p38α was added to a rapidly stirring cuvette to induce the dissociation of inhibitor. Acrylodan fluorescence was monitored at 468 nm. Under these conditions, the koff of BIRB-796 (left) and MG001 (right) were measured to be 4.54 x 10-5 s-1 and 1.08 x 10-2 s-1, respectively.

**Figure 6**

Binding of BIRB-796 and MG001 to ac-p38α and direct measurement of kon. BIRB-796 or MG001 was mixed with ac-p38α (0.1 μM) in various ratios (1-4:1 inhibitor:protein). Acrylodan fluorescence was monitored at 468 nm following the addition of inhibitor to a rapidly stirred cuvette. Under these conditions, the kobs of BIRB-796 (left) and MG001 (right) were measured at each dose and used to determine kon values of 4.46 x 103 M-1 s-1 and 9.27 x 103 M-1 s -1, respectively.

**Figure 7**

Binding of BIRB-796, staurosporine and SB203580 to ac-p38α. The high affinity ATPcompetitive inhibitor of p38α, SB203580, binds with a Kd ~15 nM while staurosporine is not detected (left). Each inhibitor was incubated with 50 nM ac-p38α overnight to obtain the data for binding curves. For real-time kinetic measurements, a single dose of each inhibitor (10 μM) was added to ac-p38α (0.1 μM). ATP-competitive inhibitors produce an instantaneous change in fluorescence (right). Weaker binding ATP-competitive inhibitors (Kd >20 nM) induce smaller changes (smaller magnitude) or no change at all (not shown).

**Figure 8**

Binding of BIRB-796 to ac-p38α in different HTS formats. BIRB-796 was incubated with acp38α overnight at 4oC. In 96-well plates, 1 nM - 2 μM inhibitor was used while 10 nM - 20 μM inhibitor was used in 384-well plates. Under these conditions, the Kd of BIRB-796 was ~27 nM in a 96-well format (left) and ~76 nM in a 384-well format (right).

**Figure 9**

Binding experiment of BIRB-796 and ac-p38α to determine time-dependent inhibition. The protein ligand mixture was incubated for 24 hours at 4˚C with fluorescence measurements taken at various time intervals Plotted binding curves reveal the expected time-dependence of BIRB-796 inhibition.

**Figure 10**

Core structure of compounds in the used DFG-out library. The proposed binding mode of these compounds is also shown (left) and orientated with BIRB-796 for comparison (right). Regions extending into the ATP site and allosteric site are variable.

**Figure 11**

Characterization of a DFG-out compound library hit. The structure of 85-C8 is shown (a) with the conserved moiety shared by all hits highlighted (red=. Using the cuvette method, 100 nM ac-p38alpha was incubated overnight with 10-100 μM of 85-C8 and emission spectra were collected (B) and binding curves were generated (C). Real-time fluorescence measurements were also performed by monitoring emission of acrylodan at 468 nm and adding a single dose of 85-C8 (5-30 μM) (D). Addition of these compounds resulted in a mixed fluorescence response with an initial rapid fluorescence change followed by slow first-order decay. Both the fast (E) and slow (F) phases are dose-dependent.

**Figure 12**

Using the ac-p38α assay to predict the binding mode of a DFG-out compound library hit. The binding mode of structurally similar compounds (A), dasatinib and INH-29, is predominantly ATP-competitive as a result of H-bonding to the hinge region of the kinase. Alignment of 87-F9 (orange) with dasatinib (magenta) reveals strong conservation of several H-bond donors or acceptors in the drug scaffold which interact with the hinge region of the kinase (B). The long extension of the library hit structure may allow it to enter the allosteric pocket (C). Dasatinib lacks this feature and only produces an instantaneous fluorescence change when added to ac-p38α, indicative of totally ATP-competitive binding. The binding modes of dasatinib INH- 29 are adapted from Andersen et al. (6).

**Figure 13**

Amino acid sequence alignment of the activation loops of several kinases. Residues are colored according to their similar properties; hydrophobic/non-charged (red), polar/acidic (blue), polar/basic (pink), polar/uncharged (green). The length of the loop appears at the end of each sequence. Specific motifs are boxed in, labeled and described in the text. The labeling position is marked **(*)** according to the position chosen for p38α. The first half of the activation loop closest to the DFG motif is the shortest in length in p38α. Structural information for other kinases which have longer activation loops reveals that the residue directly following the DFG motif **(#)** aligns well with the site chosen for ac-p38α.

**Figure 14**

Structural alignment of kinases with p38α for determining the fluorophore attachment site. cAbl kinase was aligned with active (A) and inactive (B) p38α to reveal the conformations adopted by the lengthy activation loop and suggests that the first position after the DFG motif is best for labeling in such kinases. This residue is positioned most similarly to the labeled site in p38α, which is one residue farther away from the DFG motif. In EGFR, the positioning in the DFG-out conformation was used to select a unique position which is much farther from the DFG motif (C). After formation of the unique inactive state of EGFR, this residue is positioned most similar to the labeled site of inactive p38α.

**Figure 15**

Co-crystal structure of ac-p38α in complex with Type II inhibitor sorafenib. Electron density maps of sorafenib (pink) and acrylodan (white) are contoured at 11σ. Possible hydrogen bonding interactions are highlighted by dotted lines (a). Structural alignment of the ac -p38α-sorafenib complex with the b-Raf-sorafenib complex.

The examples illustrate the invention

**Example 1: Selection of a Suitable Kinase**

We chose to work with p38α to develop this assay for the following reasons: i) the abundance available of structural information, ii) the availability of crystal structures in both its active and inactive conformations (Figure 1A.) and iii) the availability of tight binding Type II & III allosteric inhibitors. In the first step, the crystal structures of p38α were closely examined to identify suitable fluorophore attachment sites that would detect allosteric binders. Candidate residues for this mutation must be solvent exposed to enable the attachment of a fluorophore by Michael addition, and exhibit significant movement upon ligand binding. Care was also taken to not choose residues that are critical to maintaining protein stability, catalytic activity or residues in the vicinity of known phosphorylation sites.
A position near the N-terminal end of the activation loop was selected and subsequently mutated into a cysteine residue (Figure 1B.,C.). Acrylodan was selected as the fluorophore due to its relatively small size (comparable to a tryptophan side chain), its high sensitivity to polarity changes, its commercial availability and relatively low price. Acrylodan is also known to produce a robust response and should detect movements of the activation loop upon binding of allosteric inhibitors (Figure 1D.). Before labeling the protein, it was necessary to reduce the chances of fluorophore attachment to any other solvent exposed cysteine residues. Again, structural information was used to locate 4 reduced cysteine residues in p38α. Two of these cysteine are buried within the protein while the other two were solvent-exposed and conservatively mutated into serine. Lastly, a F327L mutation was incorporated to partially activate (Askari et al., 2007: Avitzour et al., 2007) the acrylodan-labeled p38α (ac-p38α) for use in enzyme activity assays, if desired.

**Example 2: Protein Labeling and Fluorescence Characterization**

<u>Protein labeling</u>

An N-terminal GST-p38α construct containing 4 total mutations (2 cysteine 4 serine, and the introduction of a cysteine for labeling) was transformed into the BL21(DE3) *E. coli* strain, overexpressed, purified by affinity, anion exchange and size exclusion chromatography and the pure protein was subsequently used for labeling. Protein and free acrylodan were combined at a 1:1.5 ratio and allowed to react in the dark overnight at 4˚C. The conjugated protein (ac-p38α) was concentrated, aliquoted and frozen at -20˚C. Mono-labeling of 100% of the protein was verified by ESI-MS. Confirmation of the correctly labeled cysteine is currently being performed by analyzing the tryptic fragments of unlabelled and labeled p38α following a combination of HPLC and ESI-MS or MALDI.

<u>Fluorescence characterization</u>

Following labeling, the fluorescent properties of the probe were characterized and initial experiments were carried out using various derivatives of the pyrazolo-urea Type II allosteric inhibitor, BIRB-796 (Pargellis et al., 2002; Dumas et al., 2000 (a and b); Moss et al., 2007; Regan et al., 2002; Regan et al., 2003). The ac-p38α protein labeled on the activation loop shows a strong red-shift from 468 nm to 514 nm with ligand binding (Figure 2). A large change at 468 nm allows for the possibility of making single-wavelength measurements. However, measuring a ratio of two wavelengths (R = 514 nm / 468 nm) allows the possibility of eliminating dilution errors between different samples. Using these two wavelengths, the normalized intensity change compared to average intensity ($\Delta I_{std}$) was determined to be 0.50 and the maximum standard intensity change ($\Delta R_{max}$) between saturated and unsaturated ac-p38α was 1.24. These are two of the most important criteria for fluorescence spectroscopy (de Lorimier et al., 2002) and both values together with a Z factor of 0.80 characterize this as a suitable probe for use in fluorescence assays. All further work presented below refers to ac-p38α tagged on the activation loop.
This labeling strategy was also applied to a position on the P-loop of p38α, but the fluorescence response of this probe was not characterized as ideal for use in a screening assay for allosteric inhibitors. However, there is some evidence in the data suggesting that this probe may provide useful information about the equilibrium between the active and inactive states for p38α in the absence of ligand. Additional experiments on this labeled protein are still underway.

**Example 3: Real-time Measurements**

Using polystyrene cuvettes (4 clear sides), real-time measurements of inhibitor binding were performed by

delivering various concentrations of BIRB-796 to a suspension of 100 nM ac-p38α. A mini stir bar was placed in the bottom of each cuvette to ensure rapid mixing as inhibitor was delivered through the injection port located above the cuvette. Following addition of the inhibitor, the fluorescence emission at 468 nm decreased in a dose-dependent manner with a first-order kinetic (Figure 3A.). These types of experiments yield rate constants ($k_{obs}$) which can be plotted and fit linearly to obtain the $k_{on}$ (slope) and $k_{off}$ (y-intercept) of each compound (Figure 3B.). The $k_{on}$ for BIRB-796 obtained using this approach ($k_{on}$ = 2.57 x $10^4$ $M^{-1}s^{-1}$) is similar to published values (Pargellis et al., 2002; Sullivan et al., 2005), while the estimated $k_{off}$ was 1-2 orders of magnitude faster ($k_{off}$ = 3.45 x $10^{-4}$ $s^{-1}$) than that measured by other methods (Pargellis et al., 2002; Sullivan et al., 2005). The conditions for such measurements are currently being further optimized (buffer, temperature, protein and inhibitor concentrations, length of incubation). Current attempts at directly measuring $k_{off}$ by adding an excess of unlabelled protein to a suspension of ac-p38α bound with inhibitor are currently underway.

**Example 4: Endpoint measurements**

Before scaling to a 384-well plate format, initial Kd measurements were carried out in cuvettes until conditions could be optimized (buffer, temperature, protein and inhibitor concentrations, length of incubation). Simple binding equilibrium experiments were carried out to determine the Kd of BIRB-796 binding to p38α. Individual cuvettes containing 50 nM ac-p38α and various concentrations of BIRB-796 (1-100 nM) were incubated at 4˚C overnight and measured 24, 48, 72 and 96 h later. We found that the Kd of BIRB-796 was time-dependent, as reported elsewhere (Pargellis et al., 2002), which necessitates longer incubation times for Type II inhibitors. All Type III inhibitors required only an overnight incubation.

The emission spectrum of each sample was measured and the fluorescence ratio (R) was calculated and plotted to show the saturation of ac-p38α in the inactive state (Figure 3C.) or plotted on a logarithmic scale to determine the Kd (Figure 3D.). Similar experiments were carried out for a focused pyrazolo-urea library of 15 compounds synthesized in the group with varying affinities for the allosteric site of p38α. The compounds and their Kd values are listed in Table 1. Kd values determined using this probe vary as much as 10-fold from published values ((Pargellis et al., 2002; Dumas et al., 2000 (a and b); Moss et al., 2007; Regan et al., 2002; Regan et al., 2003; Sullivan et al., 2005) with the largest differences occurring for compounds with a published Kd of < 10 nM. However the Kd values follow the same trend as found in the literature. Although lowering the concentration of ac-p38α in the assay would likely improve the values obtained for the tightest binding compounds, a concentration of 50 nM probe has been determined to be the lower limit that can be used to obtain reproducible data with high signal-to-noise. It is also worthy to note that all published Kd values for these compounds are calculated from rate constants ($k_{off}/k_{on}$) and not measured directly.

**Example 5: Extension of endpoint measurements to further compounds**

Several additional Type II inhibitors were tested using endpoint measurements to obtain the Kd of binding to p38α. The most important feature of these compounds is that they do not share the pyrazolourea scaffold of our numerous other compounds which were used to initially characterize the assay. This was a crucial step towards demonstrating that the change in fluorescence is dependent only on the change in protein conformation and not on the drug scaffold which is bound.

Of particular importance are the results obtained for the drugs lapatinib (Tykerb) and imatinib (Gleevec), selective potent Type II inhibitors of EGFR and Abl/PDGFR kinases, respectively. Addition of these compounds to ac-p38α did not result in a fluorescence change or measureable Kd for either compound. However, addition of Sorafenib (Nexavar), a well-known bRaf and VEGFR2 inhibitor, produced a strong fluorescence response indicative of allosteric binding to p38α. The data obtained for these compounds is shown in Figure 4.

Table 1: Measured $K_d$ values of pyrazolourea derivatives of BIRB-796

| Compound | Kd [nM] | Compound | Kd [nM] |
|---|---|---|---|
| 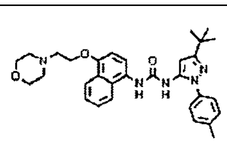 | 5 | 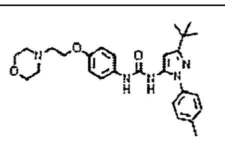 | 419 |
| 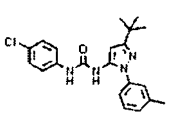 | 15 | 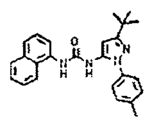 | 11 |

(continued)

| Compound | Kd [nM] | Compound | Kd [nM] |
|---|---|---|---|
| 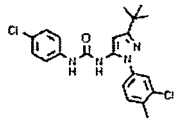 | 18 | 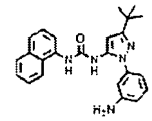 | 12 |
| 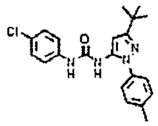 | 34 | 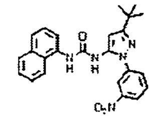 | 3 |
| 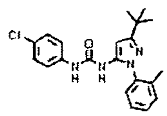 | 347 | 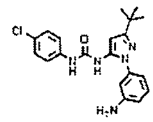 | 55 |
| 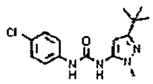 | 1,190 | 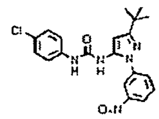 | 19 |
| 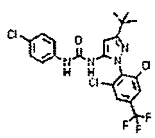 | No Binding | 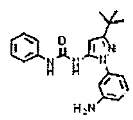 | 197 |
| | | 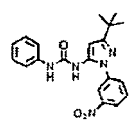 | 163 |

In a recent publication by scientists at Ambit Biosciences, 38 known kinase inhibitors were screened against a panel of 317 kinases and Kd values were measured in an attempt to quantitate inhibitor binding to off-target kinases (Karaman et al., 2008). They found that lapatinib and imatinib do not bind to p38α, while sorafenib binds with a Kd ~ 370 nM. Sorafenib was the first allosteric compound of another drug scaffold to validate this assay. The Kd of sorafenib was found to be time-dependent, similar to other Type II inhibitors, resulting in Kd values of 115 nM and 56 nM after 6 and 24 hr incubation times, respectively. These values are similar to the published Kds for sorafenib against its intended kinase targets, bRaf and VEGFR2. The higher Kd value obtained in the Ambit study for binding to p38α is likely the result of the standard conditions of their screen in which inhibitors and protein were only incubated for 1 hr.

**Example 6: Reversibility of Fluorescence - Effect of ATP & Inhibitor Dissociation**

Since the DFG-in and DFG-out conformations of kinases are believed to be a dynamic equilibrium, it was important to demonstrate the reversibility of the fluorescent change observed in the presence of allosteric binders. We have obtained titration curves for MG001 in the presence and absence of intracellular concentrations of ATP (5 mM). MG001 was chosen since it is the weakest allosteric binder in our compound collection and likely to be competed out of the kinase by high concentrations of ATP, which would shift the kinase more towards the DFG-in conformation. As expected, the binding curve of MG001 was significantly affected by the presence of ATP, resulting in a higher measured Kd of 1.62 μM.
Another set of measurements was then attempted to demonstrate the reversibility of the fluorescence change by inducing inhibitor dissociation. After allosteric binders were added to and allowed to equilibrate with ac-p38α, a 10-fold excess of non-labeled p38α was added to the cuvette while monitoring the fluorescence of acrylodan at 468 nm. The addition of excess kinase causes the inhibitor to redistribute, resulting in a net dissociation of inhibitor from ac-p38α and a fluorescence increase which was fit to a first-order function. A 10-fold excess of unlabeled kinase is a standard protocol used to ensure that the rate of dissociation would reflect the true $k_{off}$ from the protein (Hibbs et al., 2004). Adding smaller

amounts of unlabelled kinase would likely not force the dissociation of inhibitor as effectively, resulting in artificially slower dissociation rates. Normally, addition of an allosteric inhibitor results in a fluorescence decrease in the case of ac-p38α (see previous report). The dissociation of BIRB-796 and MG001 are shown in Figure 5.

These measured $k_{off}$ values are different from those published by Pargellis et al. by a factor of 10 for both BIRB-796 and MG001 (Pargellis et al., 2002). More specifically, the rate of dissociation for MG001 is 10-fold faster in our assay while that of BIRB-796 is 10-fold slower. We believe that these differences are a consequence of the type of assay used by Pargellis et al., in which the dissociation of pyrazolourea compounds is measured by using p38α-specific ATP competitive inhibitor, SKF86002, as a displacer. Upon binding, SKF86002 becomes fluorescent thereby providing a way to monitor BIRB-796 dissociation. However, this inhibitor has a Kd ~180 nM (Pargellis et al., 2002) and would therefore more effectively compete with MG001 (published Kd ~1.16 μM) than BIRB-796 (published Kd ~0.1 nM) by shifting the activation loop toward the DFG-in conformation. Since Pargellis et al., calculate Kd from $k_{on}$ and $k_{off}$, the inefficient displacement of BIRB-796 by SKF86002 would result in a lower apparent Kd since calculated Kd values are more subject to the conditions under which the rate constants are obtained.

**Example 7: Kinetics - Determination of $k_{on}$**

After measuring $k_{off}$ for allosteric compounds, we established conditions for measuring the $k_{on}$ of the same compounds. This was accomplished using the cuvette method by adding various concentrations of inhibitor to ac-p38α and fitting the fluorescence decay to a first-order function. The observed rate constant ($k_{obs}$) of the fluorescence decay for a specific dose of inhibitor was then plotted against the inhibitor concentration. Under the established conditions, inhibitor was added in molar equivalents to ac-p38α (1-4:1 inhibitor:protein) and the result is typically a straight line which can be fitted linearly with a $R^2 > 0.99$. These conditions are similar to those used elsewhere for determining $k_{on}$ of a ligand to a protein with a single binding site (Hibbs et al., 2004). The slope of this line gives $k_{on}$ for the binding of the inhibitor to the kinase. The determination $k_{on}$ for BIRB-796 and MG001 is shown in Figure 6.

These measured $k_{on}$ values are different from those published by Pargellis et al. by a factor of 100 for both BIRB-796 and MG001 (Pargellis et al., 2002). However, as reported in that study, the kon for these two inhibitors are very similar to each other. The differences in their Kd values is attributed primarily to differences in $k_{off}$, as we also observed and described above.

Interestingly, we were also able to perform the SKF86002 displacement assay for BIRB-796, but were unable to duplicate the $k_{on}$ values obtained by Pargellis et al. However, using similar conditions to our ac-p38α assay (amount of protein and inhibitor, buffer, temperature and mixing conditions) and using the same ratios of p38α to SKF86002, we obtained a $k_{on}$ with from the SKF86002 assay of 1.00 x 103 M-1s -1, which is very well comparable to the value obtained using ac-p38α.

Using the rate constants for binding and dissociation that were measured directly using ac-p38α, we obtained a calculated Kd for BIRB-796 and MG001 of 10.2 nM and 1.16 μM, which are in strong agreement with the values we obtain through endpoint measurements under similar conditions. By reaching similar results through two different methods, we are confident that the fluorescent-labeled kinase approach can not only provide accurate Kd measurements, but also valuable kinetic information about binding and dissociation from the protein.

**Example 8: Detection of Potent ATP-competitive inhibitors/identifying False Hits in Screens**

In addition to several allosteric inhibitors, we have tested several known ATP competitive inhibitors of p38α in our assay. The majority of compounds, including ATP, did not generate any kind of fluorescence change upon binding to ac-p38α. However, in the case of the most potent inhibitors (Kd ~1-20 nM), the fluorescence change was robust, allowed binding curves to be generated and the resulting Kd values were comparable to the published values for binding to p38α. However, compounds which bind to p38α with a Kd >20nM, the Kd values measured in our assay begin to diverge quickly from the published values. A few examples are shown in Table 2.

A simple experiment was designed to confirm that the fluorescent-tagged kinase loses the ability to accurately sense the binding of ATP-binding compounds with Kd >20nM.

We used the ATP-competitive inhibitor described above, SKF86002, to make endpoint measurements and obtain a binding curve with a Kd ~78 nM using the intrinsic fluorescence of the inhibitor which is produced upon binding to the ATP-binding site of ac-p38α. This value is actually slightly lower than the published value of 180 nM (Pargellis et al., 2002). Since the fluorescence of SKF86002 is measured at 420 nm, there was no interference from acrylodan in these measurements. Using an alternative approach, we used the same protein and inhibitor samples and generated a titration curve based on acrylodan fluorescence. In this case, the Kd value obtained was 10-fold higher (Kd ~721 nM). Therefore, our assay not only detects all allosteric binders which induce the DFG-out conformation of the kinase, it can also report the Kd for some tight binding ATP-competitive inhibitors. An interesting finding was that the promiscuous ATP-competitive kinase inhibitor staurosporine was not detected by our assay. In fact, it was reported that p38α is one

of the few kinases that staurosporine does not inhibit (Karaman et al., 2008).

Table 2. ATP-competitive inhibitors tested in the ac-p38α assay.

| Name | Compound | Kd (nM) | Pub. Kd. (nM) | Ref. |
|---|---|---|---|---|
| SB203580 | 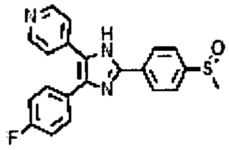 | 15 | 9-20 | |
| Dasatinib | 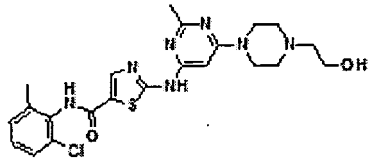 | 389 | 27 | |
| SKF86002 | 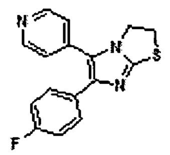 | 721 | 180 | |
| Ro3201195 | 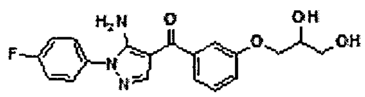 | 1,914 | 95-170 | |
| Staurosporine | 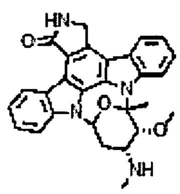 | Not Detected | >10000 | |

We are currently looking at structural information to understand how the fluorescent labeled kinase can sense these compounds, particularly when there is no conformational change in the activation loop and the inhibitor is not close enough to the fluorophore to directly alter its fluorescence. The most likely explanation is that some ATP-competitive inhibitors induce another kind of conformational change in kinases upon binding which could cause a slight shift or rotation in the position of the acrylodan without affecting the activation loop. The slightest shift into a more polar or charged environment could be enough to change the fluorescence. In particular, we will continue to examine structures to determine how the Kd of SB203580 is accurately reported by ac-p38α, with a special focus on ATP competitive compounds which may form an interaction with the Asp of DFG or compounds that approach or enter the small hydrophobic sub-pocket in the vicinity of the gatekeeper residue of p38α. These structural investigations are close to completion. As a result of these findings, it is likely that a few ATP-competitive inhibitors may register as false hits while screening for allosteric inhibitors. Therefore, it was necessary to determine whether or not ATP and allosteric compounds could be discriminated from one another using our assay.

Using the cuvette method, we were quickly able to accomplish this by looking at the kinetics of the fluorescence changes. In the case of allosteric inhibitors such as BIRB-796, we have already shown that the kinetic is relatively slow and the fluorescence change takes several minutes to reach completion. However, in the case of ATP-competitive inhibitors such as SB203580, the induced fluorescence change is instantaneous (2-4 sec). This is not surprising since a protein conformational change is not required to allow binding of these compounds as is the case for allosteric inhibitors. This instantaneous response was also observed for Ro3201195 and SKF86002. An example of these results is shown in Figure 7.

**Example 9: 384-well Plate Format**

The endpoint assay described for cuvettes for measuring the Kd of allosteric inhibitors has now been scaled down for use in a 384-well and 96-well plate formats with 20 μl and 100-200 μl total drop volumes for these plate types, respectively. Care was taken to improve inhibitor solubility and to limit the number of dilution and pipetting steps. Results for BIRB-796 are shown in Figure 8.
For 384-well plates, inhibitor stocks were prepared in DMSO at 20X the final desired concentration. Each well contained 1 μl of inhibitor solution + 19 μl of buffer containing 50 nM ac-p38α (5% v/v DMSO after mixing). The buffer is the same as that used in the cuvette method with the addition of 0.01% v/v Brij-35, a standard detergent used to improve inhibitor solubility. Under these conditions, no visible precipitation of BIRB-796 was observed. At this time, repeated screens have been performed with the inhibitor BIRB-796 to optimize the signal-to-noise ratio, incubation time and incubation temperature. After mixing, an incubation time of 6 hrs at room temperature or overnight at 4oC was found to be adequate to reach equilibrium and achieve the lowest measureable Kd for each inhibitor (Kd ~ 70 nM for BIRB-796) and good signal-to-noise (Z factor ~0.77). Using the cuvette method described above, we were able to demonstrate the expected time-dependence of BIRB-796 inhibition of p38α (Figure 9).
Similar buffer and incubation conditions were used in a 96-well format. However, 200X stocks of inhibitors were prepared in DMSO and diluted into a total volume of 200 μl (0.5% v/v DMSO after mixing). In this format, the Kd for BIRB-796 was found to be ~27 nM with slightly better signal-to-noise ratios than the 384-well format (Z factor ~0.84). All measurements of the plates were made with a Tecan Safire2.
Compared to the values obtained with the cuvette method, the measured Kds are 5-fold higher for the tightest binding inhibitor, BIRB-796, in the 96-well format and even higher in the 384-well format. Therefore, the HTS format most suitable for Kd estimation is the 96- well format while 384-well plates are the best for initial HTS screens of allosteric binders.

**Example 10: Application of HTS-format**

Since our initial report, the 384-well format has been used to make endpoint measurements in a compound library screen. The molecules screened were designed using computer simulations and modeling, to bind to the DFG-out conformation of kinases. The DFG-out conformation is the conformation required for allosteric inhibitor binding which is detected by this assay. The core structure of these compounds and their proposed binding mode is shown in Figure 10. All compounds share a 2,5-disubstituted thiazole moiety with a urea or amide in the 2 position to generate similar interactions with the kinase as the classic p38α pyrazolurea compounds. The thiazole moiety was designed to be positioned near the small hydrophobic sub-pocket into which the naphthalene moiety of BIRB-796 is bound in p38α and result in the proper positioning of the amide or urea moiety to make the characteristic interactions made by pyrazolourea compounds for strong binding to this pocket. Similarly, bulky hydrophobic moieties were placed after the urea/amide position to better occupy the allosteric pocket. The opposite end of the molecules were decorated with various alkyl moieties and/or phenyl rings along with polar hydroxyl groups, amines and N atoms which could form H-bonding interactions in the more polar adjacent ATP-binding pocket.
Compounds of varying size were generated to create a library of potential Type III (exclusively allosteric) and Type II (bridged between the allosteric and ATP sites) binders.

For this screen, a pipetting scheme was generated in which 4 dilutions of each library compound were prepared in 384-well plates using DMSO as the solvent. Each dilution was 20 X the desired final concentrations (0.05, 0.5, 5, 50 μM) used in the screen. The pipetting scheme for the assay was as described above for 384-well plates with the exception that the amount of ac-p38α was increased to 100 nM to avoid any background fluorescence from the compounds which may be present at high concentrations (500 μM). BIRB -796 was used as positive allosteric binding control and ac-p38α without inhibitor was used for background/baseline fluorescence. After mixing, the plates were incubated at RT for 5-6 hrs before measurement with a Tecan Safire2.
The screen identified 11 compounds which all increased the fluorescence ratio of ac-p38α at a concentration of 500 μM. This corresponds to a 1.8% hit rate. However, only 5 of these hits bound stronger than the remaining 7 compounds. No compound generated a fluorescence change as significant as that of BIRB-796, suggesting that all hits are weaker binding compounds. Interestingly, the structures of these compounds shared a similar feature in the region of the molecule which was proposed to bind in the ATP site, leading us to propose that the binding mode of these compounds is actually flipped 180° from the proposed mode. T his particular moiety may give the compounds a favorable interaction with the protein and may induce the DFG-out conformation and the fluorescence response.
The next step was to verify the screening results and to assess the binding mode of these compounds (ATP-competitive vs. allosteric) as described above using the cuvette method. All hits produced the correct changes in emission spectra (Figure 2) and the 5 strongest hits had Kds ranging between 14-40 μM. The remaining hits from the large library screen had Kds of 40-60 μM. It is important to note that the Kd values for these compounds are rather high, which is likely due to the fact that each compound has a stereocenter and was present as an enantiomeric mixture in the library as provided

by the manufacturer. Enantiomerically pure hits would very likely have a lower Kd. However, despite this effect, the assay was still able to identify hits which share similar structural features. Characterization of one compound (85-C8) is shown in Figure 11.

Addition of each hit to ac-p38α produced a new and surprising kind of fluorescence response which resembles a mixture of the responses seen in Figure 7 by SB203580 and BIRB-796. Upon addition of the compound, there is an instantaneous decrease in fluorescence, which is indicative of binding in the ATP pocket, followed by a slow fluorescence decay that can be fit with a first order function, which is indicative of movement of the activation loop and access to the allosteric site. Although unexpected, the magnitude of the instantaneous fluorescence change and the kinetics of the slow phase of fluorescence change are both dose-dependent and can be fit linearly when plotted against inhibitor concentration.

Given these interesting fluorescence results, we returned to the idea described above about whether or not these hits actually have a flipped binding mode. A recent publication provided us with evidence that this flipped binding mode is certainly possible (Andersen et al., 2008). In Aurora kinase, the binding mode of dasatanib (Sprycel), a Src/Abl kinase inhibitor, and INH-29 are predominantly ATP-competitive and bind to the hinge region of the kinase. Binding to this region is a prerequisite to strong ATP-competitive inhibition of kinases. Both compounds, INH-29 in particular, share many structural features with our library hits. Both are 2,5-disubstituted thiazoles and INH-29 also has a urea moiety in the 2 position. Using this crystal structure of dasatinib, we modeled in on of the library hits and overlaid it onto dasatinib and found great alignment of many pharmacophore N atoms in the structures. This structure allowed us to construct a model of the proposed binding mode of our hits, in which the conserved chemical moiety of these compounds is just long enough to extend from the ATP binding site into the allosteric pocket. The comparison of our hits with dasatinib is shown in Figure 12.

Thus, the data combined with our models suggest that the hits bind rapidly to the hinge region of the kinase in a manner similar to dasatinib. Once bound, the conserved structural moiety of the hit compounds might slowly position itself in part of the allosteric pocket and trigger the slow fluorescence change which follows the initial rapid response.

Real-time fluorescence measurements of each of the strongest hit compounds seem to support this idea *(data not shown)*. Addition of a single dose (30 μM) of each hit compound to a cuvette containing ac-p38α results in fast fluorescence changes of variable magnitudes. However, the $k_{obs}$ of the slow phase of fluorescence change is in a similar range for all hit compounds (4.2 - 8.8 x 10-4 M-1s-1). Dasatinib lacks a moiety which can reach the allosteric pocket when bound in this mode, and in our cuvette assay, dasatinib only produces an instantaneous fluorescence response which is characteristic of exclusively ATP-competitive inhibitors. Interestingly, titration of dasatinib with ac-p38α resulted in a Kd ~389 nM. This is much higher than the reported Kd of ~27 nM in p38α (Karaman et al., 2008), again suggesting that most of the inhibitor resides in the ATP pocket and is in line with our observations that acp38α can only accurately report the Kd of ATP-competitive inhibitors with Kd <20nM.

**Example 11: Labeling Strategy; Selection of a Labeling Site Using Sequence of Structural Data**

In order to effectively demonstrate the feasibility of this approach in other kinases, we have selected a series of Serine/Threonine and Tyrosine kinases from various species as the next candidates for this assay system. Most importantly, we have chosen a series of kinases which are known to be regulated by the DFG-in and DFG-out conformational switch and for which some structural information is available either in one or both conformations. We have also chosen kinases for which it is still unkown whether or not they can adopt the DFG-out conformation. For these kinases (GSK3β (human), GSK3 (fungal homolog), cSrc, CSK, EGFR (human), Lck (human) and Aurora A (human)), sequence alignments of the activation loop were generated using the DFG motif and a highly conserved APE motif as the start and end points of the loop, respectively. We also aligned the sequences of kinases for which no structural information is yet available (DDR1 (human) and pfMAPK1 (Plasmodium *falciparum*)).The alignment of these kinases is shown in Figure 13.

In p38α, we attached the fluorophore two positions after the DFG motif (occupied by an alanine) by mutating this position, which is often a highly conserved alanine or serine, with a cysteine.

This site was chosen because it sits between the highly conserved DFG motif (Box 1 of Figure 13) and the remainder of the activation loop, which contains numerous potential phosphorylation sites and various charged and/or hydrophobic residues involved in organizing the tertiary structure of the loop. All attempts were made to avoid these regions of the loop while also keeping in mind that the largest fluorescence changes will come from distinct changes in environment (solvent accessibility) rather then the quantitative distance of fluorophore movement. We will perform SASA (surface area solvent accessibility) calculations for the fluorophore provided structural information has been obtained for ac-p38α in both the DFG-in and DFG-out conformations. Thus far, we have observed the fluorophore only in the DFG-out conformation.

The labeling position chosen for p38α is typically followed by, in most kinases, a basic amino acid such as Lys or Arg (Box 2) that is involved in either forming ionic interactions with helix C of the kinase or interacts with phosphorylated

residues in other regions of the activation loop. In most kinases, this position is frequently followed by a few hydrophobic residues such as Leu or Ile (Box 3), then a few more charged residues involved in stabilizing the activation loop (Box 4), then a variable phosphorylation region containing serine, Thr or Tyr residues (Box 5). Near the end of activation loop, a highly conserved basic residue followed by one or two bulky hydrophobic planar residues such as Tyr and Trp, can be found (Box 6). This is usually followed by the highly conserved APE motif (Box 7) at the end of the loop.
Analysis of these alignments reveals that p38α has a particularly short (more compact) activation loop compared to most kinases. Therefore, structural information was more helpful in identifying the most analogous labeling position in other kinases. For each kinase aligned above, available structures were aligned with the active (PDB code: 1wbo) and inactive (PDB code: 1wbs) form of p38α. In all cases, the extra long activation loops reveal that the position labeled in p38α may not be the best position for kinases which have a longer activation loop. For these kinases, the position directly following the DFG motif, which is one position before the p38α labeling site, appears to align best structurally. In most kinases, a Leu is found in this position. In cases where no structural information is available, as with DDR1 and pfMAPK1, structural models based on known structural kinase templates were generated using online tools to assist with the identification of the labeling site and cysteine residues which are solvent exposed. Mutation of these cysteines into serine is critical to eliminating non-specific fluorescent labeling. This kind of information cannot be obtained easily by looking at a sequence alignment.

Other exceptional cases also exist, such as Aurora A kinase which has a Trp residue immediately following the DFG motif. In this case, the Trp residue does not appear to change position significantly during the transition to the DFG-out conformation. Given the planar ring structure of the fluorophore, the adjacent position in the amino acid sequence was also avoided for labeling to help prevent favorable or unfavorable interactions with the hydrophobic Trp residue. Thus, the third position following DFG was chosen in Aurora A (Val).
Another exceptional case is EGFR, which forms an alternate inactive state and does not seem to be regulated by the DFG switch. Inactive EGFR undergoes a conformational change of the activation loops which brings it more into the ATP binding site where it forms a mini α-helix. Although it may not be possible to screen for allosteric inhibitors in such a kinase, we are attempting to use the same principals to label this kinase and screen for compounds which might induce this inactive conformation. Given the unique nature of EGFR, sequence alignments alone would not be enough to determine the best labeling site. Therefore, structural information was used to identify the position on the activation loop of EGFR which would relocate to a position similar to the labeled site of p38α in the DFG-out conformation. We determined this to be a Leu which is five residues after the DFG motif. Several structural alignments are shown in Figure 14 to illustrate these points.
Aside from a few exceptions, it seems the first two residues after the DFG motif are optimal for labeling the vast majority of kinases for use in this assay approach.

**Example 12: Crystal structure of ac-p38α in complex with sorafenib**

To better understand the atomic and molecular basis of the described assay principle, we set out to crystallize acrylodan labeled p38α in presence and absence of Type I, Type II and Type III kinase inhibitors. So far, we solved the crystal structure of ac-p38α in complex with sorafenib up to a resolution of 2.5 A (Figure 15). The kinase is found in its inactive state with the activation loop adopting the DFG-out conformation. Positive difference densities for the acrylodan labeled C172 and the Type II inhibitor Sorafenib are clearly visible. The fluorophore is found in an hydrophobic environment sandwiched between F168 (DFG-motif) and the P-loop. The halogenated phenyl moiety of the inhibitor occupies the allosteric binding pocket that is only present when the kinase is in its inactive conformation. Hydrogen bonding interactions between the urea moiety of the inhibitor and the side chain of E71 (helix C) and the backbone NH of D168 (DFG-loop) are clearly indicated and in accord with the previously reported b-Raf sorafenib complex (PDB-code 1 uwh (Wan et al. Cell 2004)). The substituted pyridine binds to the hinge region of the kinase. Interestingly, the orientation of this pyridine ring is significantly different compared to the b-Raf complex. Additionally, the hinge region around M109 shows at least two conformations. At his point, we cannot rule out that the observed differences in the binding mode of sorafenib and the changes in the hinge region are somehow associated with fluorophore labeling of the protein. Co-crystallization experiments of unlabeled p38α in complex with sorafenib as well as for BIRB-796 in acrylodan labeled and unlabeled p38α are underway.

**Example 13: Application of Different Fluorophores**

We have recently completed initial tests of p38α labeled with a selection of three different thiol-reactive fluorophores, depicted in Table 3 which are reported to be sensitive to environmental changes. For detection of changes in the activation loop conformation, we chose to examine whether these fluorophores would also be suitable for this assay approach.
The fluorophore attachment was carried out as described for acrylodan. Initial fluorescence measurements were then

made to determine the optimal excitation and emission wavelengths. Real-time fluorescence measurements were then attempted using the emission maxima for each fluorophore. A single dose of 0.1 μM sorafenib was added to a cuvette containing 0.1 mM of each newly labeled p38α individually. A binding kinetic similar to that obtained under the same conditions with acrylodan-labeled p38α was obtained in all cases.
The performance of NBD-p38α was superior to the other two fluorophores, generating an increase in fluorescence upon movement of the activation loop. A similar response was observed for fluorescein-p38α, but with poorer signal-to noise. Pyrene-p38α produced a decrease in fluorescence upon inhibitor binding. Of the three new fluorophores, NBD-p38α had the highest sensitivity at the wavelength measured. Fluorescein-p38α also appeared to give good enough sensitivity for use with this method. However, the signal-to-noise for pyrene-p38α was poor and would likely not be suitable for this approach. We are also planning to test other fluorophores including Atto680 and IAEDANS.

Table 3: Thiol-reactive fluorophores tested in the fluorescent kinase assay. The structures of pyrene, fluorescein and NBD (iodoacetamide) derivatives) are shown with acrylodan for comparison. NBD and acrylodan are relatively small in size while pyrene and fluorescein are considerably more bulky.

| pyrene | fluorescein | NBD | acrylodan |
| --- | --- | --- | --- |
| 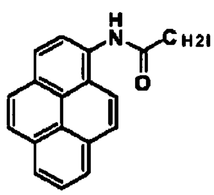 | 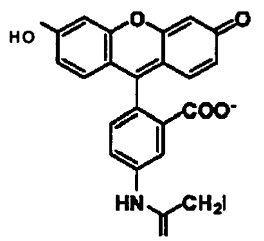 | 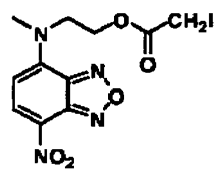 | 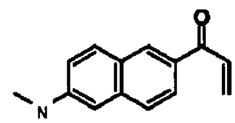 |

More experiments must be performed to determine the $\Delta I_{std}$ for each fluorescent-labeled p38α. Determination of $\Delta R_{max}$ will not be possible since these fluorophores exhibit only changes in intensity. No shift in the emission maxima were observed for NBD, fluorescein or pyrene as is observed for acrylodan. Thus, this highlights the point that the criteria for both fluorescent parameters (described in the first report) are not necessary for the development of an assay. As long as one of these criteria is met, the fluorophore-kinase conjugate has a reasonably high chance for success in this assay.
Lastly, given the structural information obtained for sorafenib bound to ac-p38α, it is not surprising that the smallest fluorophores (acrylodan and NBD) provide the best results. The packing of the acrylodan between the P-loop and the Phe of the DFG motif (in the DFG-out conformation) may very well be the reason why the fluorescence response of acrylodan is so sensitive and this interaction may be a key to the success of this approach although this is speculative. Looking at the structures of the fluorophores, this structural feature may explain why NBD performs better than other bulkier fluorophores. Therefore, it is likely that the use of smaller fluorophores would be more likely to succeed in this assay. This may also apply to any adaptation of this approach to NMR through the attachment of small isotope (i.e. 13C) enriched molecules or spin labels which would exhibit a change in chemical shift upon changes in the conformation of the activation loop.

**References**

Andersen, C.B., et al., Discovery of selective aminothiazole aurora kinase inhibitors. ACS Chem Biol, 2008. 3(3): p. 180-92.

Annis, D. A., Nazef, N., Chuang, C. C., Scott; M. P., and Nash, H. M. (2004) A general technique to rank protein-ligand binding affinities and determine allosteric versus direct binding site competition in compound mixtures, Journal of the American Chemical Society 126, 15495-15503.

Askari, N., Diskin, R., Avitzour, M., Capone, R., Livnah, O., and Engelberg, D. (2007) Hyperactive variants of p38alpha induce, whereas hyperactive variants of p38gamma suppress, activating protein 1-mediated transcription, The Journal of biological chemistry 282, 91-99.

Avitzour, M., Diskin, R., Raboy, B., Askari, N., Engelberg, D., and Livnah, O. (2007) Intrinsically active variants of all human p38 isoforms, The FEBS journal 274, 963-975.

Caputo GA, London E. Cumulative effects of amino acid substitutions and hydrophobic mismatch upon the transmembrane stability and conformation of hydrophobic alpha-helices. Biochemistry. 2003 Mar 25;42(11):3275-85.

de Lorimier, R. M., Smith, J. J., Dwyer, M. A., Looger, L. L., Sali, K. M., Paavola, C. D., Rizk, S. S., Sadigov, S., Conrad, D. W., Loew, L., and Hellinga, H. W. (2002) Construction of a fluorescent biosensor family, Protein Sci 11, 2655-2675.
Dumas, J., Hatoum-Mokdad, H., Sibley, R., Riedl, B., Scott, W. J., Monahan, M. K., Lowinger, T. B., Brennan, C., Natero, R., Turner, T., Johnson, J. S., Schoenleber, R., Bhargava, A., Wilhelm, S. M., Housley, T. J., Ranges, G. E., and Shrikhande, A. (2000) 1-Phenyl-5-pyrazolyl ureas: potent and selective p38 kinase inhibitors, Bioorganic & medicinal chemistry letters 10, 2051-2054.
Dumas, J., Sibley, R., Riedl, B., Monahan, M. K., Lee, W., Lowinger, T. B., Redman, A. M., Johnson, J. S., Kingery-Wood, J., Scott, W. J., Smith, R. A., Bobko, M., Schoenleber, R., Ranges, G. E., Housley, T. J., Bhargava, A., Wilhelm, S. M., and Shrikhande, A. (2000) Discovery of a new class of p38 kinase inhibitors, Bioorganic & medicinal chemistry letters 10, 2047-2050.
Gauglitz, G. and Vo-Dinh, T. (2003). Handbook of spectroscopy. Wiley-VCH. Hibbs, R. E., Talley, T. T., and Taylor, P. (2004) Acrylodan-conjugated cysteine side chains reveal conformational state and ligand site locations of the acetylcholine-binding protein, The Journal of biological chemistry 279, 28483-28491.
Johnson, L. N. and Lewis, R. J. (2001)). Structural basis for control by phosphorylation. Chem. Rev. 101, 2209-2242; 40
Johnson, L. N., Noble, M. E. M. and Owen, D. J. (1996) Active and inactive protein kinases : structural basis for regulation. Cell 85, 149-158.
Lakowicz, J. R. (1999). Principles of Fluorescence Spectroscopy. Kluwer Academic / Plenum Publishers
Levinson, N.M., Seeliger, M.A., Cole, P.A. and Kuriyan J. (2008). Structural basis for the recognition of c-Src by its inactivator Csk. Cell 134; pp.124-134.
Moss, N., Breitfelder, S., Betageri, R., Cirillo, P. F., Fadra, T., Hickey, E. R., Kirrane, T., Kroe, R. R., Madwed, J., Nelson, R. M., Pargellis, C. A., Qian, K. C., Regan, J., Swinamer, A., and Torcellini, C. (2007) New modifications to the area of pyrazole-naphthyl urea based p38 MAP kinase inhibitors that bind to the adenine/ATP site, Bioorganic & medicinal chemistry letters 17, 4242-4247.
Ohren, J. F., Chen, H., Pavlovsky, A., Whitehead, C., Zhang, E., Kuffa, P., Yan, C., McConnell, P., Spessard, C., Banotai, C., Mueller, W. T., Delaney, A., Omer, C., Sebolt-Leopold, J., Dudley, D. T., Leung, I. K., Flamme, C., Warmus, J., Kaufman, M., Barrett, S., Tecle, H., and Hasemann, C. A. (2004) Structures of human MAP kinase kinase 1 (MEK1) and MEK2 describe novel noncompetitive kinase inhibition, Nature structural & molecular biology 11, 1192-1197.
Pargellis, C., Tong, L., Churchill, L., Cirillo, P. F., Gilmore, T., Graham, A. G., Grob, P. M., Hickey, E. R., Moss, N., Pav, S., and Regan, J. (2002) Inhibition of p38 MAP kinase by utilizing a novel allosteric binding site, Nature structural biology 9, 268-272.
W. A Pearson, Rapid and Sensitive Sequence Comparison with FASTP and FASTA, in Methods in Enzymology, ed. R. Doolittle Academic Press, San Diego) 183(1990)63-98.
Regan, J., Breitfelder, S., Cirillo, P., Gilmore, T., Graham, A. G., Hickey, E., Klaus, B., Madwed, J., Moriak, M., Moss, N., Pargellis, C., Pav, S., Proto, A., Swinamer, A., Tong, L., and Torcellini, C. (2002) Pyrazole urea-based inhibitors of p38 MAP kinase: from lead compound to clinical candidate, Journal of medicinal chemistry 45, 2994-3008.
Regan, J., Pargellis, C. A., Cirillo, P. F., Gilmore, T., Hickey, E. R., Peet, G. W., Proto, A., Swinamer, A., and Moss, N. (2003) The kinetics of binding to p38MAP kinase by analogues of BIRB 796, Bioorganic & medicinal chemistry letters 13, 3101-3104.
Rendell, D. (1987). Fluorescence and Phosphorescence. Crown
Richieri, G. V., Ogata, R. T., and Kleinfeld, A. M. (1999) The measurement of free fatty acid concentration with the fluorescent probe ADIFAB: a practical guide for the use of the ADIFAB probe, Molecular and cellular biochemistry 192, 87-94.
Sharma, A. and Schulman, S. G. (1999). Introduction to Fluorescence Spectroscopy. Wiley interscience.Sullivan, J. E., Holdgate, G. A., Campbell, D., Timms, D., Gerhardt, S., Breed, J., Breeze, A. L., Bermingham, A., Pauptit, R. A., Norman, R. A., Embrey, K. J., Read, J., VanScyoc, W. S., and Ward, W. H. (2005) Prevention of MKK6-dependent activation by binding to p38alpha MAP kinase, Biochemistry 44, 16475-16490.
Taylor, S. S. and Radzio-Andzelm, E. (1994) Three protein kinase structures define a common motif. Structure 2, 345-355; 43
Vogtherr, M., Saxena, K., Hoelder, S., Grimme, S., Betz, M., Schieborr, U., Pescatore, B., Robin, M., Delarbre, L., Langer, T., Wendt, K. U., and Schwalbe, H. (2006) NMR characterization of kinase p38 dynamics in free and ligand-bound forms, Angewandte Chemie (International ed 45, 993-997.
Yem, A. W., Epps, D. E., Mathews, W. R., Guido, D. M., Richard, K. A., Staite, N. D., and Deibel, M. R., Jr. (1992) Site-specific chemical modification of interleukin-1 beta by acrylodan at cysteine 8 and lysine 103, The Journal of biological chemistry 267, 3122-3128.

Young, P.R., et al., Pyridinyl imidazole inhibitors of p38 mitogen-activated protein kinase bind in the ATP site. J Biol Chem, 1997. 272(18): p. 12116-21.
Karaman, M.W., et al., A quantitative analysis of kinase inhibitor selectivity. Nat Biotechnol, 2008. 26(1): p. 127-32.

## Claims

1. A kinase labeled at an amino acid having a free thiol or amino group, wherein said amino acid is naturally present or introduced in the activation loop of said kinase, with

   (a) a thiol- or amino-reactive fluorophore sensitive to polarity changes in its environment; or
   (b) a thiol-reactive spin label, an isotope or an isotope-enriched thiol- or amino-reactive label

   such that said fluorophore, spin label, isotope or isotope-enriched label does not inhibit the catalytic activity and does not interfere with the stability of the kinase.

2. The kinase of claim 1, which is a serine/threonine or tyrosine kinase.

3. The kinase of claim 1 or 2, which is p38α, MEK kinase, CSK, an Aurora kinase, GSK-3beta, c-Src, EGFR, Abl, DDR1, AKT, LCK or another MAPK.

4. The kinase of any one of claims 1 to 3, wherein the amino acid to be labeled having a free thiol or amino group is cysteine, lysine, arginine or histidine.

5. The kinase of any one of claims 1 to 4, wherein one or more solvent-exposed cysteines present outside the activation loop are deleted or replaced.

6. The kinase of any one of claims 3 to 5, which is p38α and wherein a cysteine is introduced at position 172 of SEQ ID NO: 1 and preferably wherein the cysteines at position 119 and 162 of SEQ ID NO: 1 are replaced with another amino acid.

7. The kinase of any one of claims 1 to 6, wherein the thiol- or amino- reactive fluorophore is a di-substituted naphthalene compound, a coumarin-based compound, a benzoxadiazole-based compound, a dapoxyl-based compound, a biocytin-based compound, a fluorescein, a sulfonated rhodamine-based compound, Atto fluorophores or Lucifer Yellow or derivatives thereof which exhibit a sensitivity to environmental changes.

8. The kinase of any one of claims 1 to 6, wherein the thiol-reactive spin-label is a nitroxide radical.

9. A method of screening for kinase inhibitors comprising

(a) providing a kinase labeled at an amino acid having a free thiol or amino group according to any one of claims 1 to 8
(b) contacting said fluorescently or spin-labeled or isotope-labeled kinase with a candidate inhibitor;
(c) recording the fluorescence emission signal at one or more wavelengths or a spectrum of said fluorescently labeled kinase of step (a) and step (b) upon excitation: or
(c)' recording the electron paramagnetic resonance (EPR) or nuclear magnetic resonance (NMR) spectra of said spin-labeled or isotope-labeled kinase of step (a) and step (b); and
(d) comparing the fluorescence emission signal at one or more wavelengths or the spectra recorded in step (c) or the EPR or NMR spectra recorded in step (c)';

wherein a difference in the fluorescence intensity at at least one wavelength, preferably at the emission maximum, and/or a shift in the fluorescence emission wavelength in the spectra of said fluorescently labeled kinase obtained in step (c), or an alteration in the EPR or NMR spectra of said spin-labeled or isotope-labeled kinase obtained in step (c)' indicates that the candidate inhibitor is a kinase inhibitor.

10. A method of determining the kinetics of ligand binding and/or of association or dissociation of a kinase inhibitor comprising

(a) contacting a fluorescently labeled kinase according to any one of claims 1 to 8 with different concentrations of an inhibitor; or
(a)' contacting a fluorescently labeled kinase according to any one of claims 1 to 8 bound to an inhibitor with different concentrations of unlabeled kinase;
(b) recording the fluorescence emission signal at one or more wavelengths or a spectrum of said fluorescently labeled kinase for each concentration upon excitation;
(c) determining the rate constant for each concentration from the fluorescence emission signals at one or more wavelengths or the spectra recorded in step (b); or
(c1) determining the $K_d$ from the fluorescence emission signal at one or more wavelengths or the spectra recorded in step (b) for each concentration of inhibitor; or
(c2) determining the $K_a$ from the fluorescence emission signal at one or more wavelengths or the spectra recorded in step (b) for each concentration of unlabelled kinase;
(d) directly determining the $k_{on}$ and/or extrapolating the $k_{off}$ from the rate constants determined in step (c) from the signals or spectra for the different concentrations of inhibitor obtained in step (b); or
(d)' directly determining the $k_{off}$ and /or exptrapolating the $k_{on}$ from the rate constants determined in step (c) from the signals or spectra for the different concentrations of unlabeled kinase obtained in step (b); and
(e) optionally calculating the $K_d$ and/or Ka from $k_{on}$ and $k_{off}$ obtained in step (d) or (d)'.

**11.** A method of determining the dissociation or association of a kinase inhibitor comprising

(a) contacting a spin-labeled or isotope-labeled kinase according to any one of claims 1 to 8 with different concentrations of an inhibitor; or
(a)' contacting a spin-labeled or isotope-labeled kinase according to any one of claims 1 to 8 bound to an inhibitor with different concentrations of unlabelled kinase;
(b) recording the EPR or NMR spectrum of said spin-labeled or isotope-labeled kinase for each concentration of inhibitor and/or unlabelled kinase; and
(c) determining the $K_d$ from the EPR or NMR spectra recorded in step (b) for the different concentrations of inhibitor; or
(c)' determining the $K_a$ from the EPR or NMR spectra recorded in step (b) for the different concentrations of unlabeled kinase.

**12.** A method of generating a mutated kinase suitable for the screening of kinase inhibitors comprising

(a) replacing solvent exposed amino acids having a free thiol or amino group, if any, present in a kinase of interest outside the activation loop or amino acids having a free thiol or amino group at an unsuitable position within the activation loop with an amino acid not having a free thiol or amino group;
(b) mutating an amino acid in the activation loop of said kinase of interest to an amino acid having a free thiol or amino group if no amino acid having a free thiol or amino group is present in the activation loop ;
(c) labeling the kinase of interest with a thiol- or amino- reactive fluorophore sensitive to polarity changes in its environment, a thiol -reactive spin label, an isotope or an isotope-enriched thiol-or amino-reactive label such that said fluorophore, spin label, isotope or isotope-enriched label does not inhibit the catalytic activity of the kinase and/or does not interfere with the stability of the kinase;
(d) contacting the kinase obtained in step (c) with a known inhibitor of said kinase; and
(e) recording the fluorescence emission signal at one or more wavelengths or a spectrum of said fluorescently labeled kinase of step (c) and (d) upon excitation or
(e)' recording the EPR or NMR spectra of said spin-labeled kinase of step (c) and (d);
(f) comparing the fluorescence emission spectra recorded in step (e) or the EPR or NMR spectra recorded in step (e)';

wherein a difference in the fluorescence intensity at at least one wavelength, preferably at the emission maximum, and/or a shift in the fluorescence emission wavelength in the spectra of said fluorescently labeled kinase obtained in step (e), or an alteration in the EPR or NMR spectra of said spin-labeled or isotope-labeled kinase obtained in step (e)' indicates that the kinase is suitable for the screening for kinase inhibitors.

**13.** The method of any one of claims 9 to 12, wherein the kinase inhibitor binds either partially or fully to the allosteric site adjacent to the ATP binding site of the kinase.

**14.** The kinase of any one of claims 1 to 8 or the method of any one of claims 9 to 13, wherein the kinase is labeled at a cysteine naturally present or introduced in the activation loop.

**15.** The method of any one of claims 9 or 12 to 14, further comprising optimizing the pharmacological properties of a compound identified as inhibitor of said kinase.

**16.** The method of claim 15, wherein the optimization comprises modifying an inhibitor identified as inhibitor of said kinase to achieve:

a) modified spectrum of activity, organ specificity, and/or
b) improved potency, and/or
c) decreased toxicity (improved therapeutic index), and/or
d) decreased side effects, and/or
e) modified onset of therapeutic action, duration of effect, and/or
f) modified pharmacokinetic parameters (absorption, distribution, metabolism and excretion), and/or
g) modified physico-chemical parameters (solubility, hygroscopicity, color, taste, odor, stability, state), and/or
h) improved general specificity, organ/tissue specificity, and/or
i) optimized application form and route by

a. esterification of carboxyl groups, or
b. esterification of hydroxyl groups with carboxylic acids, or
c. esterification of hydroxyl groups to, e.g. phosphates, pyrophosphates or sulfates or hemi-succinates, or
d. formation of pharmaceutically acceptable salts, or
e. formation of pharmaceutically acceptable complexes, or
f. synthesis of pharmacologically active polymers, or
g. introduction of hydrophilic moieties, or
h. introduction/exchange of substituents on aromates or side chains, change of substituent pattern, or
i. modification by introduction of isosteric or bioisosteric moieties, or
j. synthesis of homologous compounds, or
k. introduction of branched side chains, or
l. conversion of alkyl substituents to cyclic analogues, or
m. derivatization of hydroxyl groups to ketales, acetales, or
n. N-acetylation to amides, phenylcarbamates, or
o. synthesis of Mannich bases, imines, or
p. transformation of ketones or aldehydes to Schiffs bases, oximes, acetales, ketales, enolesters, oxazolidines, thiazolidines

or combinations thereof.

Fig. 1

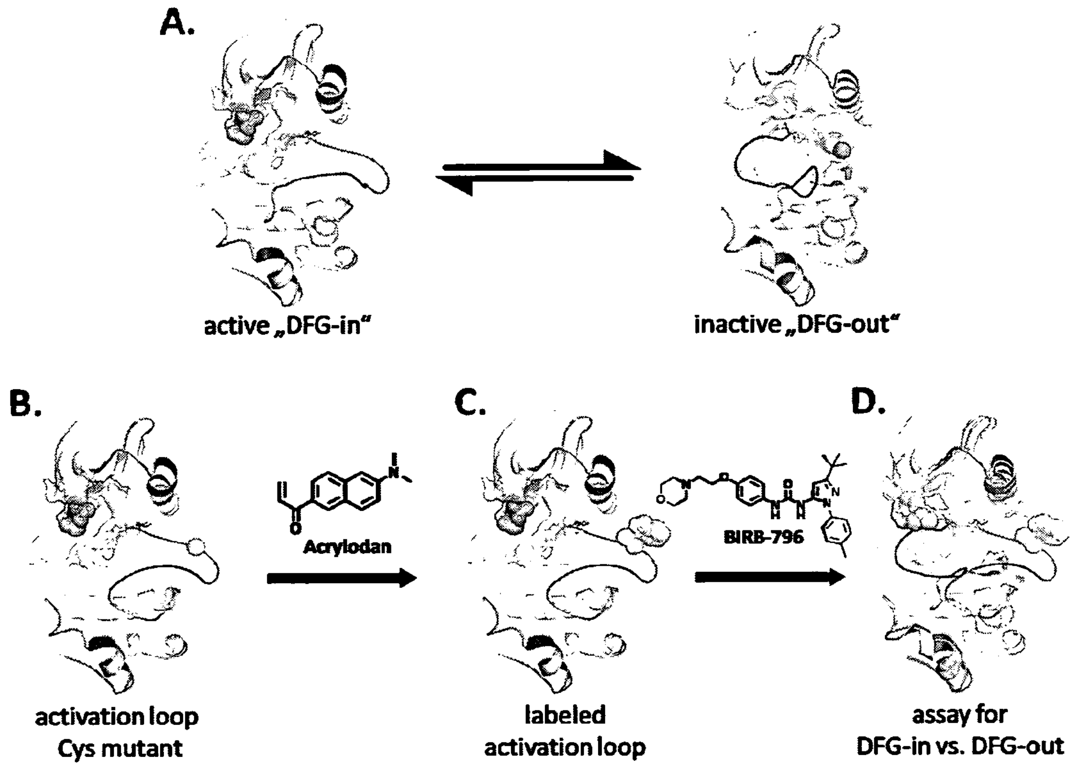
A.
active „DFG-in“
inactive „DFG-out“
B.
C.
D.
Acrylodan
BIRB-796
activation loop
Cys mutant
labeled
activation loop
assay for
DFG-in vs. DFG-out

Fig. 2

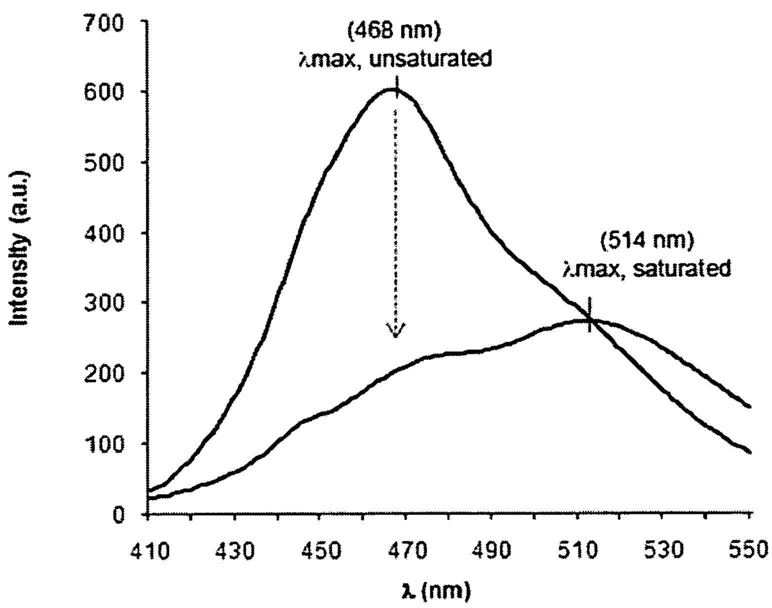
700
600
500
400
300
200
100
0
Intensity (a.u.)
(468 nm)
λmax, unsaturated
(514 nm)
λmax, saturated
410
430
450
470
490
510
530
550
λ (nm)

Fig. 3

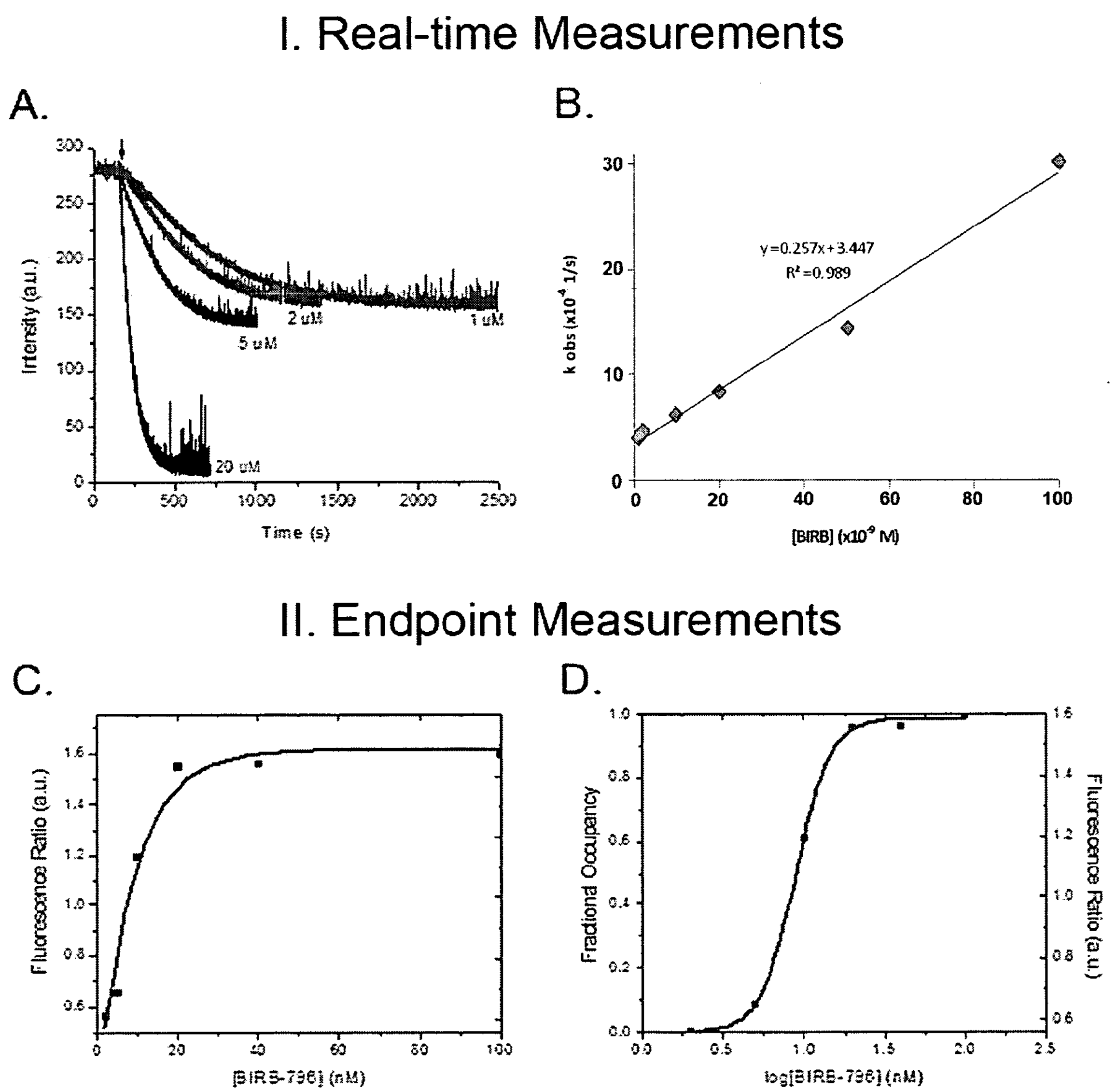
I. Real-time Measurements
A.
Intensity (a.u.)
Time (s)
5 uM
2 uM
1 uM
20 uM
B.
k obs (x10^-4 1/s)
[BIRB] (x10^-9 M)
y=0.257x+3.447
R²=0.989
II. Endpoint Measurements
C.
Fluorescence Ratio (a.u.)
[BIRB-796] (nM)
D.
Fractional Occupancy
Fluorescence Ratio (a.u.)
log[BIRB-796] (nM)

Fig. 4

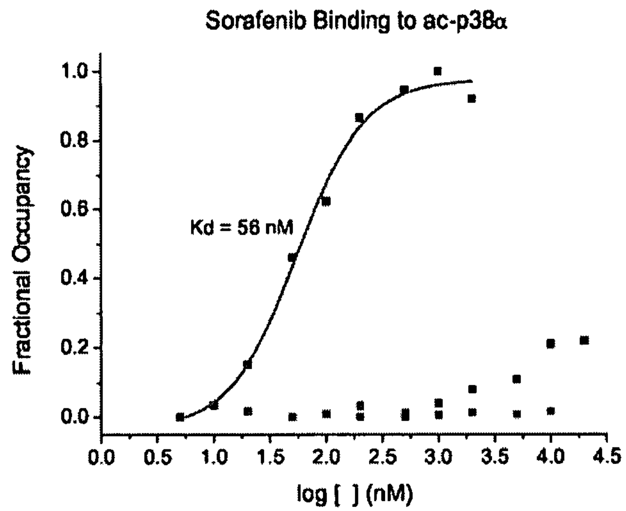


sorafenib

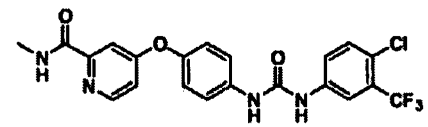

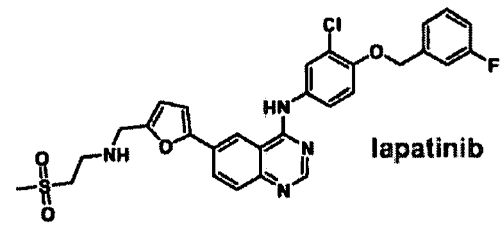

lapatinib

imatinib

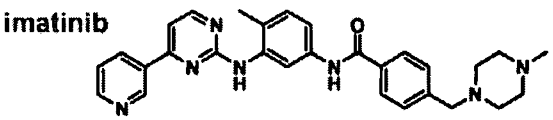

Fig. 5

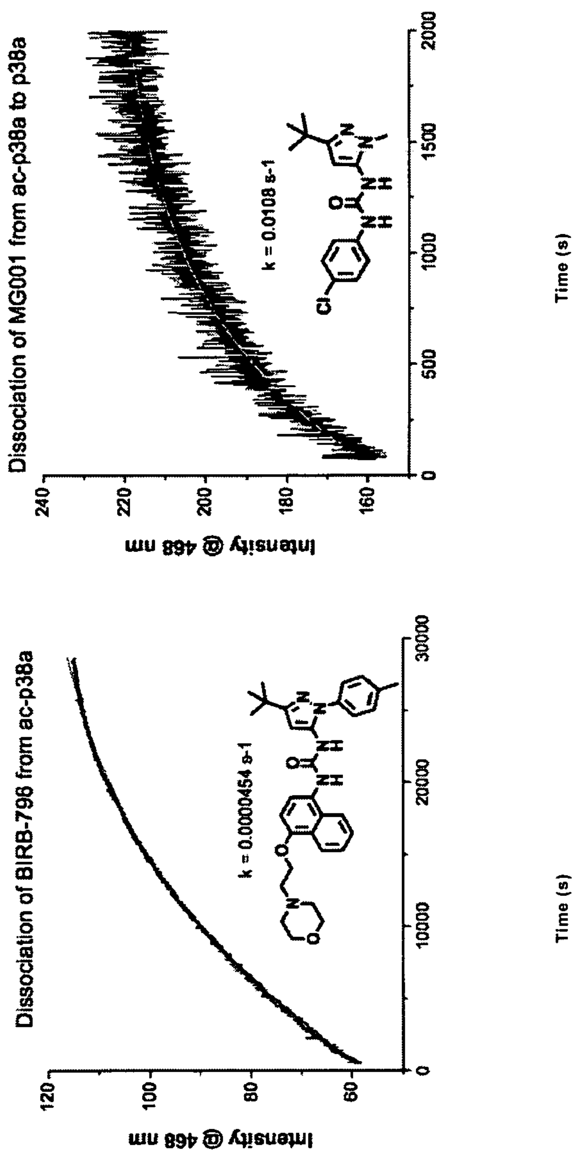

Dissociation of BIRB-796 from ac-p38a
k = 0.0000454 s-1
Intensity @ 468 nm
Time (s)
Dissociation of MG001 from ac-p38a to p38a
k = 0.0108 s-1
Intensity @ 468 nm
Time (s)

Fig. 6

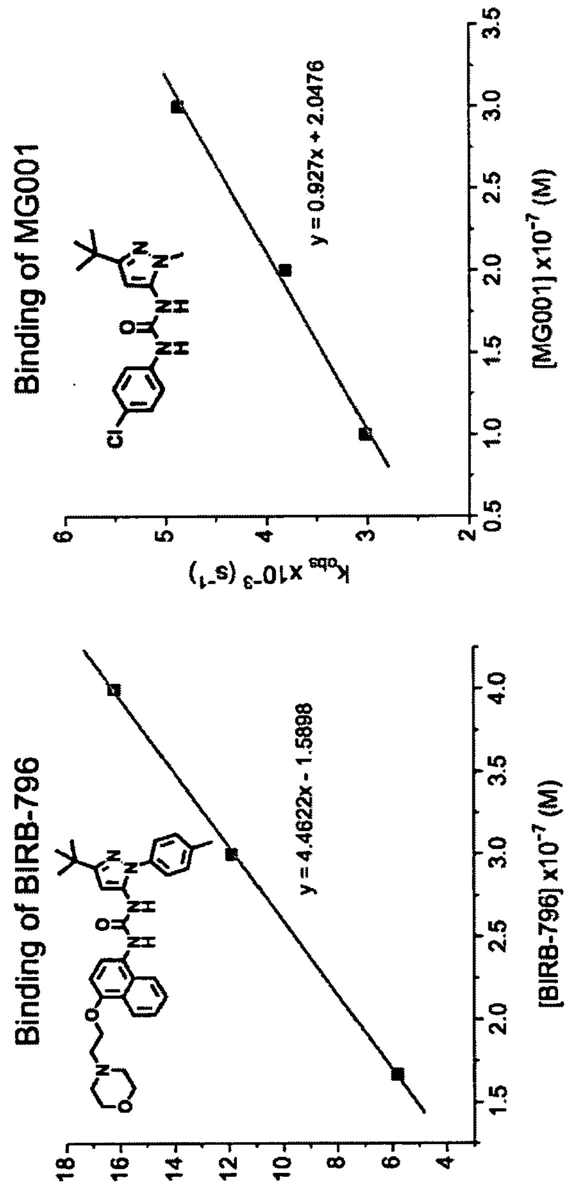
Binding of BIRB-796
y = 4.4622x - 1.5898
[BIRB-796] x10-7 (M)
kobs x10-4 (s-1)
Binding of MG001
y = 0.927x + 2.0476
[MG001] x10-7 (M)
kobs x10-3 (s-1)

Fig. 7

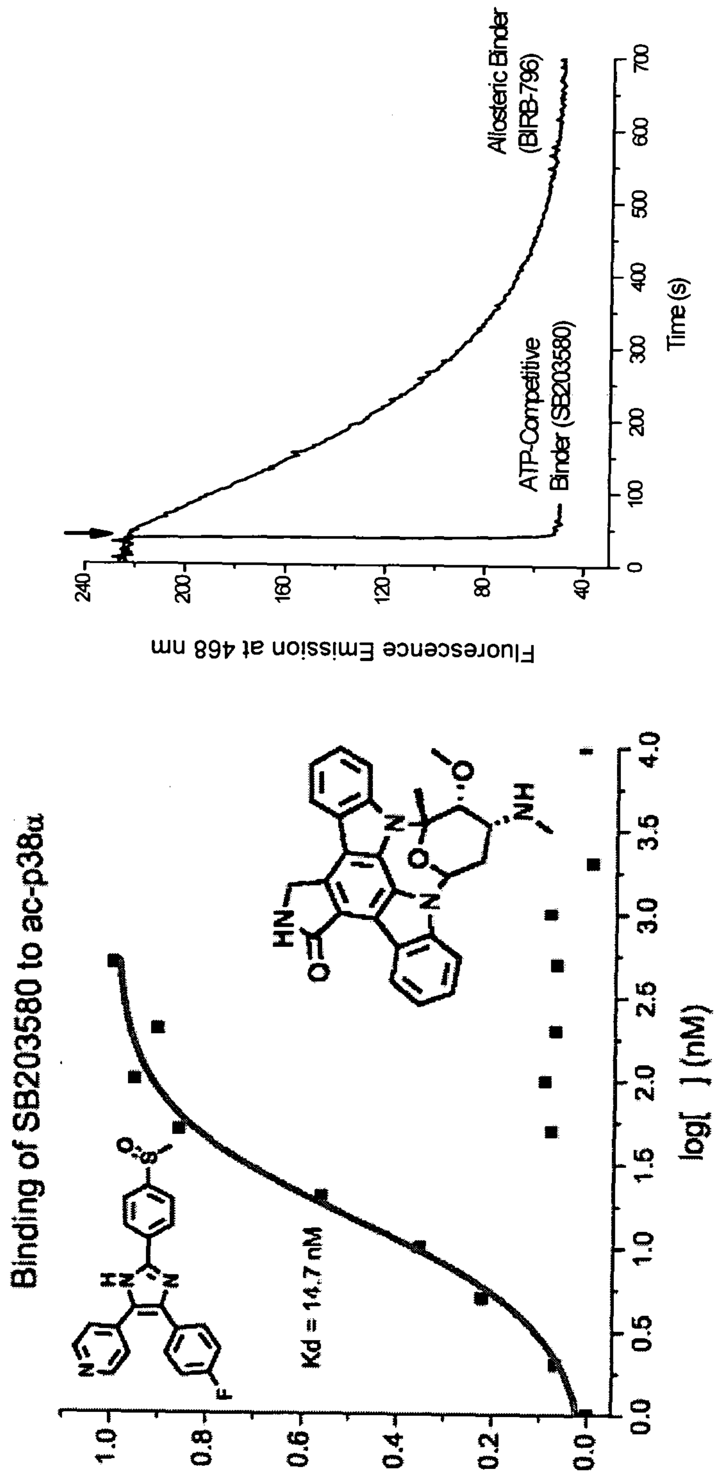
Binding of SB203580 to ac-p38α
Kd = 14.7 nM
Fractional Occupancy
0.0
0.2
0.4
0.6
0.8
1.0
0.0
0.5
1.0
1.5
2.0
2.5
3.0
3.5
4.0
log[ ] (nM)
Fluorescence Emission at 468 nm
40
80
120
160
200
240
0
100
200
300
400
500
600
700
Time (s)
ATP-Competitive Binder (SB203580)
Allosteric Binder (BIRB-796)

Fig. 8

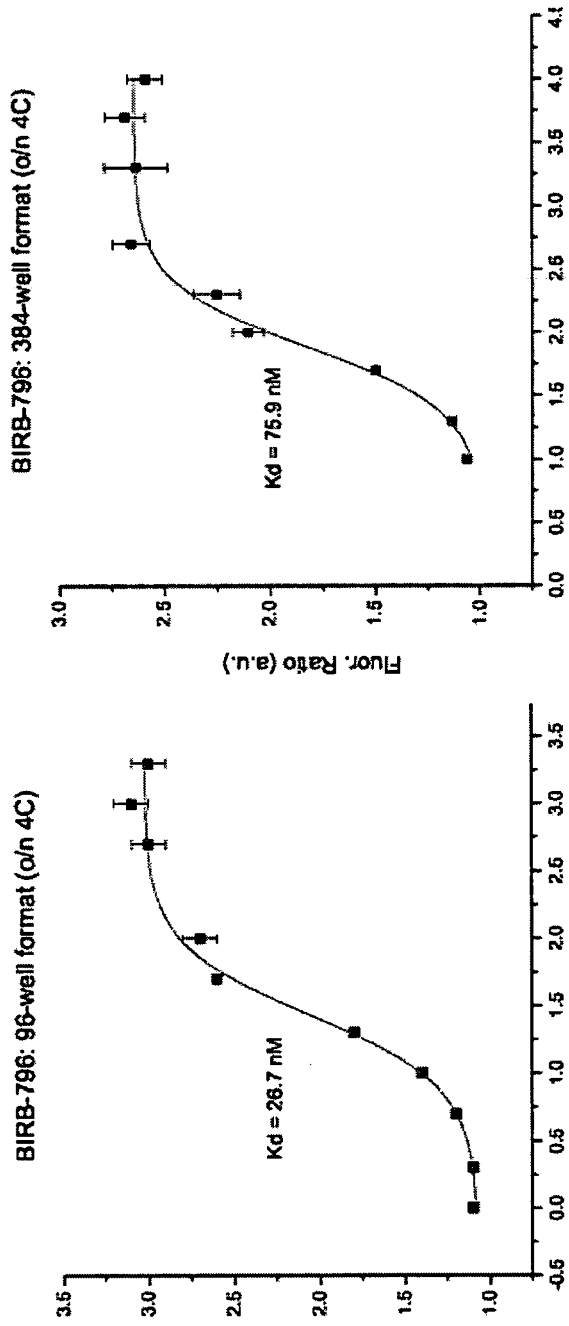
BIRB-796: 96-well format (o/n 4C)
Kd = 26.7 nM
Fluor. Ratio (a.u.)
log [ ] (nM)
BIRB-796: 384-well format (o/n 4C)
Kd = 75.9 nM
Fluor. Ratio (a.u.)
log [ ] (nM)

Fig. 9

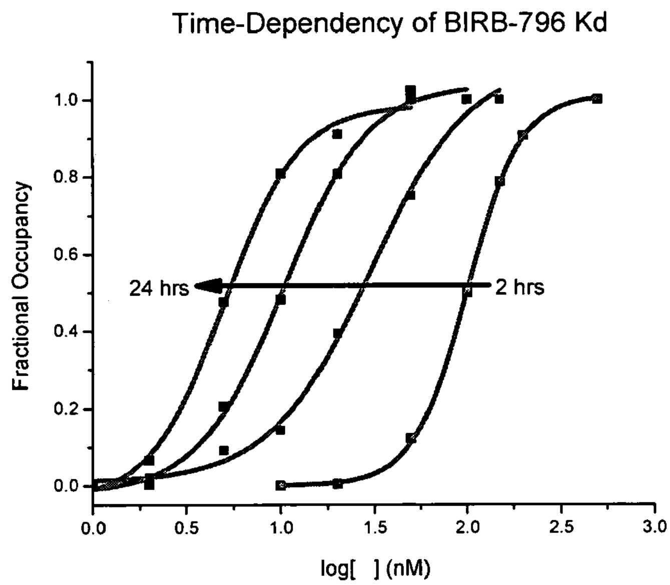
Time-Dependency of BIRB-796 Kd
Fractional Occupancy
1.0
0.8
0.6
0.4
0.2
0.0
24 hrs
2 hrs
0.0
0.5
1.0
1.5
2.0
2.5
3.0
log[ ] (nM)

Fig. 10

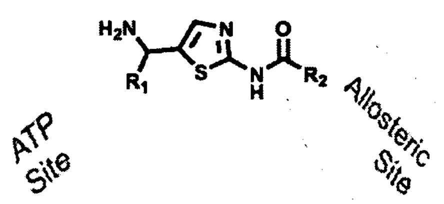
ATP Site
Allosteric Site

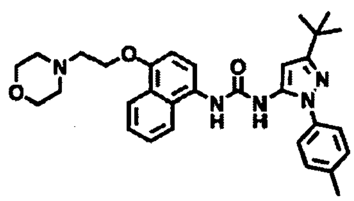

Fig. 11

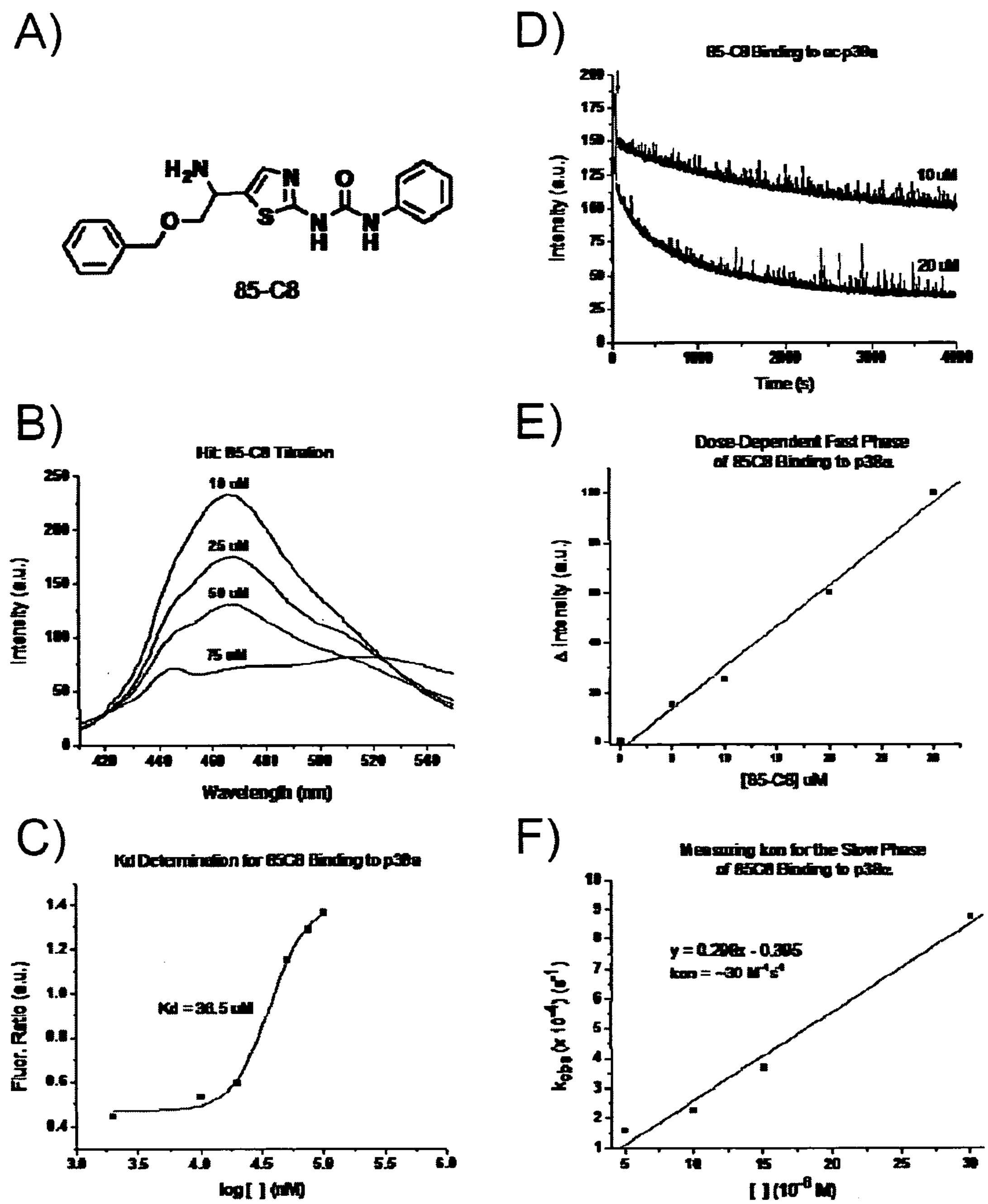
A)
85-C8
D)
Intensity (a.u.)
10 uM
20 uM
Time (s)
B)
Hit: 85-C8 Titration
10 uM
25 uM
50 uM
75 uM
Intensity (a.u.)
Wavelength (nm)
E)
Dose-Dependent Fast Phase
of 85C8 Binding to p38α
Δ Intensity (a.u.)
[85-C8] uM
C)
Kd Determination for 85C8 Binding to p38α
Fluor. Ratio (a.u.)
Kd = 38.5 uM
log [ ] (nM)
F)
Measuring kon for the Slow Phase
of 85C8 Binding to p38α
y = 0.298x - 0.395
kon = ~30 M⁻¹s⁻¹
[ ] (10⁻⁶ M)

Fig. 12

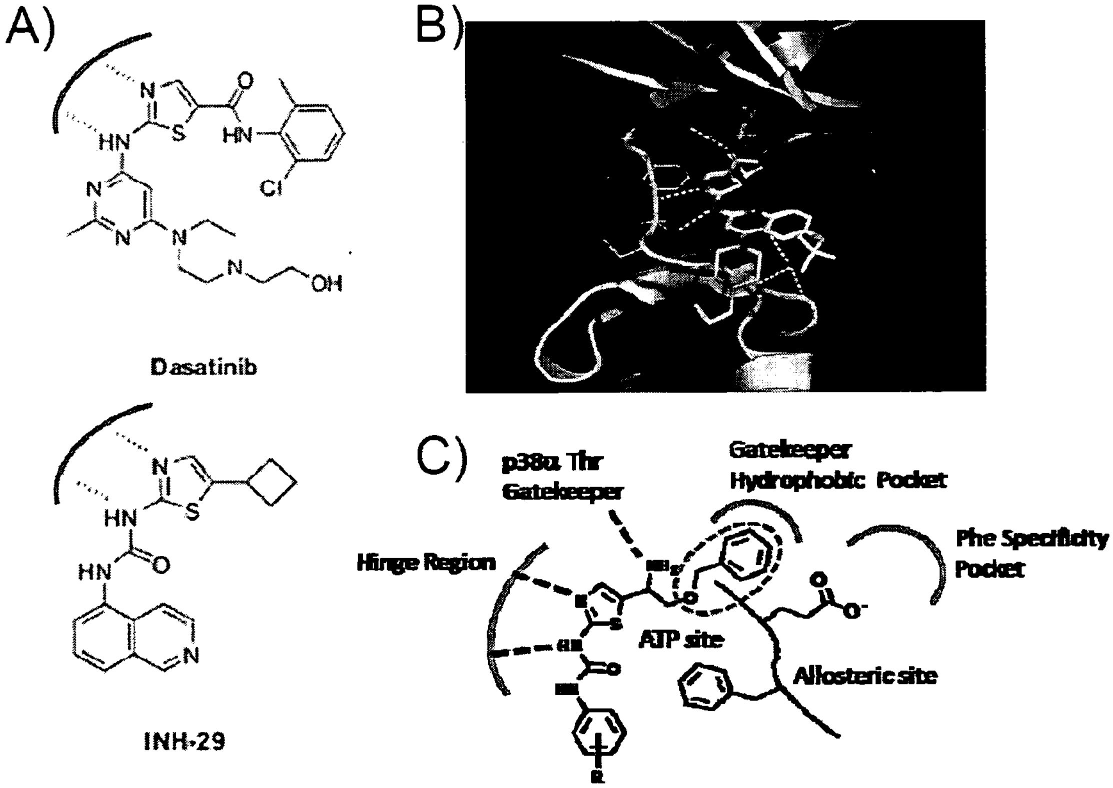
A)
B)
C)
Dasatinib
INH-29
p38α Thr Gatekeeper
Gatekeeper Hydrophobic Pocket
Phe Specificity Pocket
Hinge Region
ATP site
Allosteric site

Fig. 13

| | 1 | #* | 2 | 3 | 4 | 5 | 6 | 7 | | |
|---|---|---|---|---|---|---|---|---|---|---|
| p38a | DFG | LA | R | -- | --HTDDE | ----MTGYVAT | RWY | RAPE | --- | 25 |
| MAPK1 | DFG | LA | R | SI | STHVNEN | KVPILTDYVAT | RWY | RAPE | --- | 33 |
| GSK3beta | DFG | SA | K | -- | -QLVRGE | P---NVSYICS | RYY | RAPE | --- | 27 |
| GSK3 | DFG | SA | K | -- | -ILIAGE | P---NVSYICS | RYY | RAPE | --- | 27 |
| CSK | DFG | LT | K | -- | ----EAS | STQDTG-KLPV | KWT | -APE | --- | 25 |
| EGFR | DFG | LA | K | LL | -GAEEKE | YHAEGG-KVPI | KWM | -ALE | --- | 30 |
| cSrc | DFG | LA | R | LI | -E--DNE | YTARQGAKFPI | KWT | -APE | --- | 29 |
| Lck | DFG | LA | R | LI | -E--DNE | YTAREGAKFPI | KWT | -APE | --- | 29 |
| c-Abl | DFG | LS | R | LM | -T--GDT | YTAHAGAKFPI | KWT | -APE | --- | 29 |
| DDR1 | DFG | MS | R | NL | -Y-AGDY | YRVQGRAVLPI | RWM | -AWE | --- | 30 |
| Aurora | DFG | WS | V | -- | ----HAP | SSRRTTLCGTL | DYL | -PPE | MIE | 29 |

Fig. 14

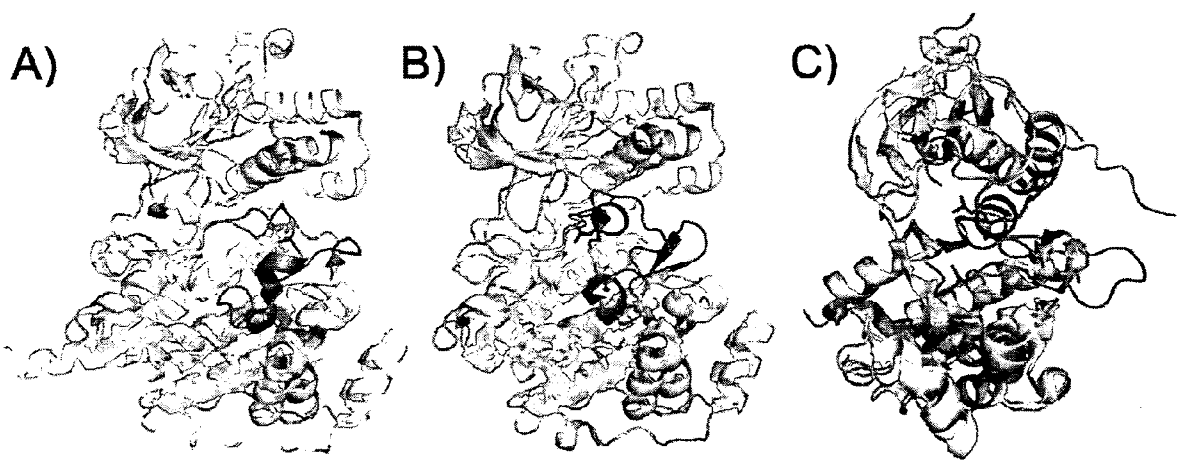

Fig. 15

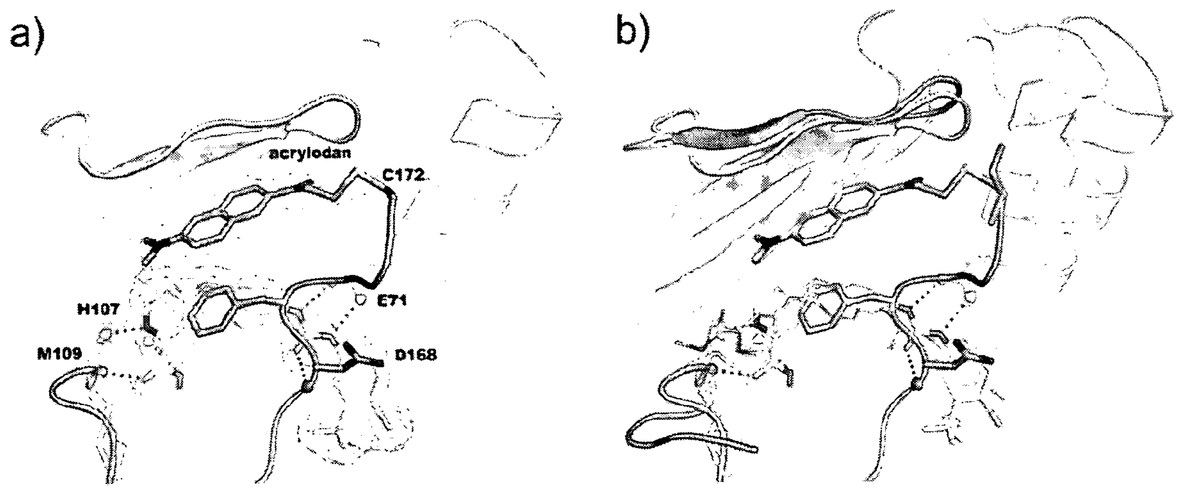

Europäisches Patentamt
European Patent Office
Office européen des brevets

# EUROPEAN SEARCH REPORT

Application Number
EP 08 01 3340

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | KAPAHI PANKAJ ET AL: "Inhibition of NF-kappaB activation by arsenite through reaction with a critical cysteine in the activation loop of IkappaB kinase" JOURNAL OF BIOLOGICAL CHEMISTRY, vol. 275, no. 46, November 2000 (2000-11), pages 36062-36066, XP002503167 ISSN: 0021-9258 * abstract * ----- | 1-16 | INV. C12Q1/48 |
| A | FIRST E A ET AL: "SELECTIVE MODIFICATION OF THE CATALYTIC SUBUNIT OF CAMP-DEPENDENT PROTEIN KINASE WITH SULFHYDRYL-SPECIFIC FLUORESCENT PROBES" BIOCHEMISTRY, AMERICAN CHEMICAL SOCIETY, EASTON, PA.; US, vol. 28, no. 8, 1 January 1989 (1989-01-01), pages 3598-3605, XP002073139 ISSN: 0006-2960 * abstract * ----- | 1-16 | |
| | | | TECHNICAL FIELDS SEARCHED (IPC) |
| A | PURI R N ET AL: "Adenosine cyclic 3',5'-monophosphate dependent protein kinase: fluorescent affinity labeling of the catalytic subunit from bovine skeletal muscle with o-phthalaldehyde." BIOCHEMISTRY 5 NOV 1985, vol. 24, no. 23, 5 November 1985 (1985-11-05), pages 6499-6508, XP002503168 ISSN: 0006-2960 * abstract * ----- -/-- | 1-16 | C12Q |

The present search report has been drawn up for all claims

1

| Place of search | Date of completion of the search | Examiner |
|---|---|---|
| The Hague | 10 November 2008 | Lunter, Pim |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 08 01 3340

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (IPC) |
| X | LANGER THOMAS ET AL: "NMR backbone assignment of a protein kinase catalytic domain by a combination of several approaches: application to the catalytic subunit of cAMP-dependent protein kinase." CHEMBIOCHEM : A EUROPEAN JOURNAL OF CHEMICAL BIOLOGY 5 NOV 2004, vol. 5, no. 11, 5 November 2004 (2004-11-05), pages 1508-1516, XP002503169 ISSN: 1439-4227 * page 1509; figure 5 * ----- | 1-16 | |
| X | MANLEY PAUL W ET AL: "Bcr-Abl binding modes of dasatinib, imatinib and nilotinib: An NMR study." BLOOD, vol. 108, no. 11, Part 1, November 2006 (2006-11), page 224A, XP002503170 & 48TH ANNUAL MEETING OF THE AMERICAN-SOCIETY-OF-HEMATOLOGY; ORLANDO, FL, USA; DECEMBER 09 -12, 2006 ISSN: 0006-4971 * abstract * ----- | 1-16 | TECHNICAL FIELDS SEARCHED (IPC) |

The present search report has been drawn up for all claims

1

| Place of search | Date of completion of the search | Examiner |
|---|---|---|
| The Hague | 10 November 2008 | Lunter, Pim |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **Holzgrabe ; Bechtold.** *Deutsche Apotheker Zeitung,* 2000, vol. 140 (8), 813-823
- **Andersen, C.B. et al.** Discovery of selective aminothiazole aurora kinase inhibitors. *ACS Chem Biol,* 2008, vol. 3 (3), 180-92
- **Annis, D. A. ; Nazef, N. ; Chuang, C. C. ; Scott; M. P. ; Nash, H. M.** A general technique to rank protein-ligand binding affinities and determine allosteric versus direct binding site competition in compound mixtures. *Journal of the American Chemical Society,* 2004, vol. 126, 15495-15503
- **Askari, N. ; Diskin, R. ; Avitzour, M. ; Capone, R. ; Livnah, O. ; Engelberg, D.** Hyperactive variants of p38alpha induce, whereas hyperactive variants of p38gamma suppress, activating protein 1-mediated transcription. *The Journal of biological chemistry,* 2007, vol. 282, 91-99
- **Avitzour, M. ; Diskin, R. ; Raboy, B. ; Askari, N. ; Engelberg, D. ; Livnah, O.** Intrinsically active variants of all human p38 isoforms. *The FEBS journal,* 2007, vol. 274, 963-975
- **Caputo GA ; London E.** Cumulative effects of amino acid substitutions and hydrophobic mismatch upon the transmembrane stability and conformation of hydrophobic alpha-helices. *Biochemistry,* 25 March 2003, vol. 42 (11), 3275-85
- **de Lorimier, R. M. ; Smith, J. J. ; Dwyer, M. A. ; Looger, L. L. ; Sali, K. M. ; Paavola, C. D. ; Rizk, S. S. ; Sadigov, S. ; Conrad, D. W. ; Loew, L.** Construction of a fluorescent biosensor family. *Protein Sci,* 2002, vol. 11, 2655-2675
- **Dumas, J. ; Hatoum-Mokdad, H. ; Sibley, R. ; Riedl, B. ; Scott, W. J. ; Monahan, M. K. ; Lowinger, T. B. ; Brennan, C. ; Natero, R. ; Turner, T.** 1-Phenyl-5-pyrazolyl ureas: potent and selective p38 kinase inhibitors. *Bioorganic & medicinal chemistry letters,* 2000, vol. 10, 2051-2054
- **Dumas, J. ; Sibley, R. ; Riedl, B. ; Monahan, M. K. ; Lee, W. ; Lowinger, T. B. ; Redman, A. M. ; Johnson, J. S. ; Kingery-Wood, J. ; Scott, W. J.** Discovery of a new class of p38 kinase inhibitors. *Bioorganic & medicinal chemistry letters,* 2000, vol. 10, 2047-2050
- **Gauglitz, G. ; Vo-Dinh, T.** Handbook of spectroscopy. Wiley-VCH, 2003
- **Hibbs, R. E. ; Talley, T. T. ; Taylor, P.** Acrylodan-conjugated cysteine side chains reveal conformational state and ligand site locations of the acetylcholine-binding protein. *The Journal of biological chemistry,* 2004, vol. 279, 28483-28491
- **Johnson, L. N. ; Lewis, R. J.** Structural basis for control by phosphorylation. *Chem. Rev.,* 2001, vol. 101, 2209-2242
- **Johnson, L. N. ; Noble, M. E. M. ; Owen, D. J.** Active and inactive protein kinases : structural basis for regulation. *Cell,* 1996, vol. 85, 149-158
- **Lakowicz, J. R.** Principles of Fluorescence Spectroscopy. Kluwer Academic / Plenum Publishers, 1999
- **Levinson, N.M. ; Seeliger, M.A. ; Cole, P.A. ; Kuriyan J.** Structural basis for the recognition of c-Src by its inactivator Csk. *Cell,* 2008, vol. 134, 124-134
- **Moss, N. ; Breitfelder, S. ; Betageri, R. ; Cirillo, P. F. ; Fadra, T. ; Hickey, E. R. ; Kirrane, T. ; Kroe, R. R. ; Madwed, J. ; Nelson, R. M.** New modifications to the area of pyrazole-naphthyl urea based p38 MAP kinase inhibitors that bind to the adenine/ATP site. *Bioorganic & medicinal chemistry letters,* 2007, vol. 17, 4242-4247
- **Ohren, J. F. ; Chen, H. ; Pavlovsky, A. ; Whitehead, C. ; Zhang, E. ; Kuffa, P. ; Yan, C. ; McConnell, P. ; Spessard, C. ; Banotai, C.** Structures of human MAP kinase kinase 1 (MEK1) and MEK2 describe novel noncompetitive kinase inhibition. *Nature structural & molecular biology,* 2004, vol. 11, 1192-1197
- **Pargellis, C. ; Tong, L. ; Churchill, L. ; Cirillo, P. F. ; Gilmore, T. ; Graham, A. G. ; Grob, P. M. ; Hickey, E. R. ; Moss, N. ; Pav, S.** Inhibition of p38 MAP kinase by utilizing a novel allosteric binding site. *Nature structural biology,* 2002, vol. 9, 268-272
- Rapid and Sensitive Sequence Comparison with FASTP and FASTA. **W. A Pearson.** Methods in Enzymology. Academic Press, 1990, vol. 183, 63-98
- **Regan, J. ; Breitfelder, S. ; Cirillo, P. ; Gilmore, T. ; Graham, A. G. ; Hickey, E. ; Klaus, B. ; Madwed, J. ; Moriak, M. ; Moss, N.** Pyrazole urea-based inhibitors of p38 MAP kinase: from lead compound to clinical candidate. *Journal of medicinal chemistry,* 2002, vol. 45, 2994-3008

- **Regan, J. ; Pargellis, C. A. ; Cirillo, P. F. ; Gilmore, T. ; Hickey, E. R. ; Peet, G. W. ; Proto, A. ; Swinamer, A. ; Moss, N.** The kinetics of binding to p38MAP kinase by analogues of BIRB 796. *Bioorganic & medicinal chemistry letters,* 2003, vol. 13, 3101-3104
- **Rendell, D.** *Fluorescence and Phosphorescence,* 1987
- **Richieri, G. V. ; Ogata, R. T. ; Kleinfeld, A. M.** The measurement of free fatty acid concentration with the fluorescent probe ADIFAB: a practical guide for the use of the ADIFAB probe. *Molecular and cellular biochemistry,* 1999, vol. 192, 87-94
- **Sharma, A. ; Schulman, S. G.** Introduction to Fluorescence Spectroscopy. Wiley interscience, 1999
- **Sullivan, J. E. ; Holdgate, G. A. ; Campbell, D. ; Timms, D. ; Gerhardt, S. ; Breed, J. ; Breeze, A. L. ; Bermingham, A. ; Pauptit, R. A. ; Norman, R. A.** Prevention of MKK6-dependent activation by binding to p38alpha MAP kinase. *Biochemistry,* 2005, vol. 44, 16475-16490
- **Taylor, S. S. ; Radzio-Andzelm, E.** Three protein kinase structures define a common motif. *Structure,* 1994, vol. 2, 345-355
- **Vogtherr, M. ; Saxena, K. ; Hoelder, S. ; Grimme, S. ; Betz, M. ; Schieborr, U. ; Pescatore, B. ; Robin, M. ; Delarbre, L. ; Langer, T.** NMR characterization of kinase p38 dynamics in free and ligand-bound forms. *Angewandte Chemie (International ed,* 2006, vol. 45, 993-997
- **Yem, A. W. ; Epps, D. E. ; Mathews, W. R. ; Guido, D. M. ; Richard, K. A. ; Staite, N. D. ; Deibel, M. R., Jr.** Site-specific chemical modification of interleukin-1 beta by acrylodan at cysteine 8 and lysine 103. *The Journal of biological chemistry,* 1992, vol. 267, 3122-3128
- **Young, P.R. et al.** Pyridinyl imidazole inhibitors of p38 mitogen-activated protein kinase bind in the ATP site. *J Biol Chem,* 1997, vol. 272 (18), 12116-21
- **Karaman, M.W. et al.** A quantitative analysis of kinase inhibitor selectivity. *Nat Biotechnol,* 2008, vol. 26 (1), 127-32

SEQUENCE LISTING

<110> Max-Planck-Gesellschaft zur Förderung der Wissenschaften e.V.

<120> Fluorescently or spin-labeled kinases for rapid screening and identification of novel kinase inhibitor scaffolds

<130> P1826 EP

<160> 1

<170> PatentIn version 3.3

<210> 1
<211> 360
<212> PRT
<213> Homo sapiens

<400> 1

Met Ser Gln Glu Arg Pro Thr Phe Tyr Arg Gln Glu Leu Asn Lys Thr
1 5 10 15

Ile Trp Glu Val Pro Glu Arg Tyr Gln Asn Leu Ser Pro Val Gly Ser
20 25 30

Gly Ala Tyr Gly Ser Val Cys Ala Ala Phe Asp Thr Lys Thr Gly Leu
35 40 45

Arg Val Ala Val Lys Lys Leu Ser Arg Pro Phe Gln Ser Ile Ile His
50 55 60

Ala Lys Arg Thr Tyr Arg Glu Leu Arg Leu Leu Lys His Met Lys His
65 70 75 80

Glu Asn Val Ile Gly Leu Leu Asp Val Phe Thr Pro Ala Arg Ser Leu
85 90 95

Glu Glu Phe Asn Asp Val Tyr Leu Val Thr His Leu Met Gly Ala Asp
100 105 110

Leu Asn Asn Ile Val Lys Cys Gln Lys Leu Thr Asp Asp His Val Gln
115 120 125

Phe Leu Ile Tyr Gln Ile Leu Arg Gly Leu Lys Tyr Ile His Ser Ala
130 135 140

Asp Ile Ile His Arg Asp Leu Lys Pro Ser Asn Leu Ala Val Asn Glu
145 150 155 160

Asp Cys Glu Leu Lys Ile Leu Asp Phe Gly Leu Ala Arg His Thr Asp
165 170 175

```
Asp Glu Met Thr Gly Tyr Val Ala Thr Arg Trp Tyr Arg Ala Pro Glu
            180                 185                 190

Ile Met Leu Asn Trp Met His Tyr Asn Gln Thr Val Asp Ile Trp Ser
        195                 200                 205

Val Gly Cys Ile Met Ala Glu Leu Leu Thr Gly Arg Thr Leu Phe Pro
    210                 215                 220

Gly Thr Asp His Ile Asp Gln Leu Lys Leu Ile Leu Arg Leu Val Gly
225                 230                 235                 240

Thr Pro Gly Ala Glu Leu Leu Lys Lys Ile Ser Ser Glu Ser Ala Arg
                245                 250                 255

Asn Tyr Ile Gln Ser Leu Thr Gln Met Pro Lys Met Asn Phe Ala Asn
            260                 265                 270

Val Phe Ile Gly Ala Asn Pro Leu Ala Val Asp Leu Leu Glu Lys Met
        275                 280                 285

Leu Val Leu Asp Ser Asp Lys Arg Ile Thr Ala Ala Gln Ala Leu Ala
    290                 295                 300

His Ala Tyr Phe Ala Gln Tyr His Asp Pro Asp Asp Glu Pro Val Ala
305                 310                 315                 320

Asp Pro Tyr Asp Gln Ser Phe Glu Ser Arg Asp Leu Leu Ile Asp Glu
                325                 330                 335

Trp Lys Ser Leu Thr Tyr Asp Glu Val Ile Ser Phe Val Pro Pro Pro
            340                 345                 350

Leu Asp Gln Glu Glu Met Glu Ser
        355                 360
```